(19) **Europäisches Patentamt / European Patent Office / Office européen des brevets**

(11) **EP 1 819 227 B1**

(12) **EUROPEAN PATENT SPECIFICATION**

(45) Date of publication and mention
of the grant of the patent:
**09.02.2011 Bulletin 2011/06**

(51) Int Cl.:
*A61K 31/7068* (2006.01)   *A61K 31/724* (2006.01)
*A61K 47/48* (2006.01)   *A61P 3/00* (2006.01)
*A61P 9/10* (2006.01)   *A61P 35/00* (2006.01)

(21) Application number: **05853560.0**

(22) Date of filing: **08.12.2005**

(86) International application number:
**PCT/US2005/044676**

(87) International publication number:
**WO 2006/071491 (06.07.2006 Gazette 2006/27)**

(54) **PHARMACEUTICAL FORMULATION OF DECITABINE**

PHARMAZEUTISCHE FORMULIERUNGEN VON DECITABIN

FORMULATION PHARMACEUTIQUE DE DECITABINE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **10.12.2004 US 9540**

(43) Date of publication of application:
**22.08.2007 Bulletin 2007/34**

(73) Proprietor: **SuperGen, Inc.
Dublin, California 94568 (US)**

(72) Inventors:
• **TANG, Chunlin
Walnut Creek, California 94596 (US)**
• **JOSHI-HANGAL, Rajashree
Pleasanton, California 94566 (US)**

(74) Representative: **Crooks, Elizabeth Caroline et al
Kilburn & Strode LLP
20 Red Lion Street
London WC1R 4PJ (GB)**

(56) References cited:
WO-A-03/103687   WO-A-2005/014010
US-A- 3 817 980   US-A- 5 874 418
US-B2- 6 613 753

• TANG CHUNLIN ET AL: "Complexation of rubitecan (9-NC) and cyclodextrins" AMERICAN CHEMICAL SOCIETY. ABSTRACTS OF PAPER. AT THENATIONAL MEETING, AMERICAN CHEMICAL SOCIETY, WASHINGTON, DC, US, vol. 227, no. Part 2, 1 March 2004 (2004-03-01), page U31, XP009101381 ISSN: 0065-7727
• THORSTEINN LOFTSSON ET AL: "Pharmaceutical Applications of Cyclodextrins. 1. Drug Solubilization and Stabilization" JOURNAL OF PHARMACEUTICAL SCIENCES, AMERICAN PHARMACEUTICAL ASSOCIATION. WASHINGTON, US, vol. 85, no. 10, 1 October 1996 (1996-10-01), pages 1017-1025, XP002080430 ISSN: 0022-3549
• SILVERMAN L. R.: 'Randomized controlled trial of azacitidine in patients with the myelodysplastic syndrome: A study of the cancer and leukemia group' JOURNAL OF CLINICAL ONCOLOGY vol. 20, no. 10, 15 May 2002, pages 2429 - 2440, XP003001920

**Description**

**FIELD OF THE INVENTION**

This invention relates generally to pharmaceutical formulations of decitabine containing a cyclodextrin compound, and methods of preparing and using the pharmaceutical formulations for treating various diseases and conditions, such as cancer and hematological disorders.

**BACKGROUND OF THE INVENTION**

A few asacytosine nucleosides, such as 5-aza-2'-deoxycytidine (also called decitabine) and 5-azacytidine (also called azacitidine), have been developed as antagonist of its related natural nucleoside, 2'-deoxycytidine and cytidine, respectively. The only structural difference between azacytosine and cytosine is the presence of a nitrogen at position 5 of the cytosine ring in azacytosine as compared to a carbon at this position for cytosine.

Two isomeric forms of decitabine can be distinguished. The β-anomer is the active form. The modes of decomposition of decitabine in aqueous solulion are (a) conversion of the active β-anomer to the inactive α-anomer (Pompon et al. (1987) J. Chromat 388:113-122); (b) ring cleavage of the aza-pyrimidine ring to form N-(formylamidine)-N'-β-D-2'-deoxy-(ribofuranosy)-urea (Mojaverian and Repta (1984) J. Pharm. Pharmacol. 36:728. 733); and (c) subsequent formulation of guanidine compounds (Kissinger and Stenm (1986) J. Chromat. 353:309-318).

Decitabine possesses multiple pharmacological characteristics. At a molecular level, it is S-phase dependent for incorporation into DNA. At a cellular level, decitabine can induce cell differentiation and exert hematological toxicity. Despite having a short half-life *in vivo*, decitabine has an excellent tissue distribution (Van Groeningen CJ, et al. (1986) Cancer Res. 46:4831-4836; and Chabot GG and Momparler RL (1990) Proceedings of the Workshop on 5-aza-2'-deoxycytidine (Momparler RL and De Vos D. eds), PCH Publication, pp. 105-115).

One of the functions of decitabine is its ability to specifically and potently inhibit DNA methylation. Methylation of cytosine to 5-methylcytosine occurs at the level of DNA. Inside the cell, decitabine is first converted into its active form, the phosphorylated 5-aza-deoxycytidine, by deoxycytidine kinase which is primarily synthesized during the S phase of the cell cycle. The affinity of decitabine for the catalytical site of deoxycytidine kinase is similar to the natural substrate, deoxycytidine. Momparler et al. (1985) 30:287-299. After conversion to its triphosphate form by deoxycytidine kinase, decitabine is incorporated into replicating DNA at a rate similar to that of the natural substrate, dCTP. Bouchard and Mompaler (1983) Mol. Pharmacol. 24:109-114.

Incorporation of decitabine into the DNA strand has a hypomethylation effect by reversal of aberrant hypermethylation characteristic of cancer as a disease. Each class of differentiated cells has its own distinct methylation pattern. After chromosomal duplication, in order to conserve this pattern of methylation, the 5-methylcytosine on the parental strand serves to direct methylation on the complementary daughter DNA strand. Substituting the carbon at the 5 position of the cytosine for a nitrogen interferes with this normal process of DNA methylation. The replacement of 5-methylcytosine with decitabine at a specific site of methylation produces an irreversible inactivation of DNA methyltransferase, presumably due to formation of a covalent bond between the enzyme and decitabine. Juttermann et al. (1994) Proc. Natl. Acad. Sci. USA 91:11797-11801. By specifically inhibiting DNA methyltransferase, the enzyme required for methylation, the aberrant methylation of the tumor suppressor genes could be prevented.

Decitabine is commonly supplied as a sterile lyophilized powder for injection, together with buffering salt, such as potassium dihydrogen phosphate, and pH modifier, such as sodium hydroxide. For example, decitabine is supplied by SuperGen, Inc., as lyophilized powder packed in. 20 mL glass vials, containing 50 mg of decitabine, monobasic potassium dihydrogen phosphate, and sodium hydroxide. When reconstituted with 10 mL of sterile water for injection, each mL contain 5 mg of decitabine, 6.8 mg of $KH_3PO_4$, and approximately 1.1 mg NaOH. The pH of the resulting solution is 6.5 - 7.5. The reconstituted solution can be further diluted to a concentration of 1.0 or 0.1 mg/mL in cold infusion fluids, i.e., 0.9% Sodium Chloride; or 5% Dextrose; or 5% Glucose; or Lactated Ringer's. The unopened vials are typically stored at room temperature (2-25°C; 32-77°F), in the original package.

Decitabine is most typically administered to patients by injection, such as by a bolus I.V. injection, continuous I.V. infusion, or I.V. infusion Similar to decitabine, azacitidine is also formulated as aqueous solution and delivered to patients intravenously. According to clinical studies of axacitidine, longer or continuous infusions were more effective than shorter ones. Santini et al. (2001) Ann. Int. Med. 134: 573-588. However, the length of I.V. infusion is limited by the decomposition/instability of decitabine or azacitidine and low solubility of the drugs in aqueous solutions. The present invention provides innovative solutions to such problems.

**SUMMARY OF THE INVENTION**

The present invention provides formulations of decitabine kits and methods of using decitabine a for preventing or treating various diseases or conditions. According to the invention, decitabine is formulated with a cyclodextrin compound, solvated in aqueous solvent comprising at least 60% v/v water. The innovative approach circumvents problems associated with the current clinical aqueous formulations of decitabine or 5-azacytidine in phosphate buffer, such as chemical instability, inconvenient storage and transportation, and discomfort of patients due to cold infusions.

In one aspect of the invention, a pharmaceutical composition is provided, comprising: decitabine and a cyclodextrin compound, preferably in solid form. The composition may further comprise buffer salt, acid, alkaline, and/or excipient. Upon dissolving in aqueous solution, the decitabine forms a complex with the cyclodextrin compound. The pharmaceutical composition may be in various dosage forms suitable for delivering, into an animal subject orally, parenterally, topically, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly, or intrathecally. The pharmaceutical composition is solvated with aqueous solvent and is suitable for administration to the subject intravenously, intramuscularly, or subcutaneously.

The decitabine may be in the form of salt, preferably pharmaceutically-acceptable salt. Examples of salts include, but are not limited to pharmaceutically-acceptable salt prepared from an inorganic acid or an organic acid. Examples of such inorganic acids are hydrochloric, hydrobromic, hydrobiotic, intric, carbonic, sulfuric and phosphoric acid. Appropriate organic acids may be selected from aliphatic, cycloaliphatic, aromatic, arylaliphatic, heterocyclic, carboxylic and sulfonic classes of organic acids, examples of which are formic, acetic, propionic, succinic, glycolic, gluconic, maleic, embonic (pamoic), methanesulfonic, ethanesulfonic, 2-hydroxyethanesulfonic, pantothenic, benzenesulfonic, toluenesulfonic, sulfanilic, mesylic, cyclohexylaminosulfonic, stearic, algenic, β-hydroxybutyric, malonic, galactic, and galacturonic acid. Preferably, the acid is selected from the group consisting of hydrochloric, L-lactic, acetic, phosphoric, (+)-L-tartaric, citric, propionic, butyric, hexanoic, L-aspartic, L-glutamic, succinic, EDTA, maleic, methanesulfonic acid, HBr, HF, HI, nitric, nitrous, sulfuric, sulfurous, phosphorous, perchloric, chloric, chlorous acid, carboxylic acid, sulfonic acid, ascorbic, carbonic, and fumaric acid. In particular, the sulfonic acid is selected from the group consisting of ethanesulfonic, 2-hydroxyethanesulfonic, and toluenesulfonic acid.

The cyclodextrin compound may be crystalline and amorphous. Preferably the cyclodextrin compound. is amorphous α-, β- or γ-cyclodextrin, more preferably alkylated or hydroxyalkyl cyclodextrin selected from the group consisting of hydroxypropyl hydroxyethyl, glucosyl, maltosyl and maltotriosyl derivatives of β-cyclodextrin, carboxyamidomethyl-β-cyclodextrin, carboxymethyl-β-cyclodextrin, hydroxypropyl-β-cyclodextrin and diethylamino-β-cyclodextrin.

Optionally, the cyclodextrin compound is a sulfoalkylether cyclodextrin derivative, preferably mono-, tetra or hepta-substituted β-cyclodextrin sulfobutyl ether sodium salt, and more preferably β-cyclodextrin sulfobutyl ether, 7 sodium salt (CAPT1SOL®).

According to the embodiment, the amount of the decitabine in the pharmaceutical formulation is between 0.1 and 200 mg per ml of solvent; optionally between 1 and 100, between 2 mg and 50 mg, 5 mg and 30 mg, between 10 mg and 25 mg per ml of the solvent

The ratio of the weight of the decitabine to the weight of cyclodextrin compound may be in a range between 1:1 and 1:5000, optionally between 1:10 and 1:300, between 1:1 and 1:200, or between 1:5 and 1:50.

The pharmaceutical formulation comprises at least 60%, 70%, 80%, 90%, 95%, 98% or 99% v/v water. The concentration of the cyclodextrin compound in the aqueous pharmaceutical formulation is between 0.1% and 80% w/w, optionally between 1% and 60% w/w, optionally between 5% and 50% w/w, optionally between 10% and 40% w/w, or optionally between 20% and 40% w/w.

Also optionally, the pharmaceutical formulation may further comprise propylene glycol, glycerin, and/or polyethylene glycol (PEG) such as PEG300, PEG400 and PEG1000.

Also according to the embodiment, the pharmaceutical composition may further comprise an acidifying agent added to the formulation in a proportion such that the formulation has a resulting pH between about 4 and 8. Adding an acidifying agent to control the pH of the formulation is believed to facilitate ready dissolution of the cytidine analog/derivative in the solvent and enhance long-term stability of the formulation.

The acidifying agent may be an organic acid. Examples of organic acid include, but are not limited to, ascorbic acid, citric acid, tartaric acid, lactic acid, oxalic acid, formic acid, benzene sulphonic acid, benzoic acid, maleic acid, glutamic acid, succinic acid, aspartic acid, diatrizoic acid, and acetic acid. The acidifying agent may also be an inorganic acid, such as hydrochloric acid, sulphuric acid, phosphoric acid, and nitric acid.

In a variation, the acidifying agents is ascorbic acid at a concentration of 0.01-0.2 mg/ml of the solvent, optionally 0.04-0.1 mg/ml or 0.03-0.07 mg/ml of the solvent.

The pH of the pharmaceutical formulation may be adjusted to be between pH 4 and pH 8, preferably between pH 5 and pH 7, and more preferably between pH 5.5 and pH 6.8.

The Pharmaceutical formulation optionally further include an excipient added in an amount sufficient to enhance the stability of the composition, maintain the product in solution, or prevent side effects (e.g., potential ulceration, vascular irritation or extravasation) associated with the administration of the inventive formulation. Examples of excipients include, but are not limited to, mannitol, sorbitol, lactose, and dextrose.

The pharmaceutical formulation is preferably at least 80%, 90% 95% or more stable upon storage at 2-8°C for 5, 10, 15, 24 hours, or 2, 4, 7, 14, 21, 28 or more days. The pharmaceutical formulation is also preferably at least 80%. 90% 95% or more stable upon storage at 20-25°C for 5, 10, 15, 24 hours, or 2, 5, 7, 14, 21, 28 or more days.

In another embodiment, the pharmaceutical formulation is prepared by the act comprising dissolving decitabine and a cyclodextrin compound in an aqueous solvent that comprises at least 60% v/v water, optionally at least 70%, 80%, 90%, 95% or 99% v/v water. The aqueous solvent may further comprise buffer salt such as potassium phosphate dissolved therein.

According to the embodiment, the act may further comprise: adding an acidifying agent to the solution containing the cytidine analog/derivative and the solvent such that pH of the resulting solution is between pH 4 and pH 8, preferably between pH5 and pH7, and more preferably between pH 5.5 and pH 6.8.

The pharmaceutical formulation may also optionally comprise one or more therapeutic agents other than decitabine, For example, the pharmaceutical formulation may optionally further comprise a therapeutic agent selected from the group consisting of anti-neoplasticagents, alkylating agents, that are members of the retinoids superfamily, hormonal agents, plant-derived agents, biologic agents, interleukins, interferons, cytokines, immuno-modulating agents, and monoclonal antibodies.

Another aspect of the invention relates to a sterilized vessel comprising a pharmaceutical formulation according to the present invention. The vessel, for example, may be a vial, syringe, or ampoule. The vessel may come in different sizes. For example, the vessel may comprise between 1 and 50, 1 and 25, 1 and 20 or 1 and 10 ml of the pharmaceutical formulation.

Yet another aspect of the invention relates to a kit comprising decitabine in a solid, preferably powder, or more preferably lyophilized powder farm, and an aqueous diluent that comprises a cycloextrin compound and at least 60% v/v water. Mixing of the solid analog/derivative and the diluent results in the formation of a pharmaceutical formulation according to the present invention.

For example, the kit may comprise a first container containing decitabine and a cyclodextrin compound in a solid, preferably powder, or more preferably lyophilized powder form; and a second container containing an aqueous diluent comprising at least 60% v/v water, wherein adding the diluent to the solid compounds results in the formation of a pharmaceutical formulation for administering the decitabine.

Optionally, the kit may comprise a first container containing decitabine in a solid form; and a second container containing a cyclodextrin compound dissolved in an aqueous diluent comprising at least 60% v/v water, wherein adding the diluent to the solid compound results in the formation of a pharmaceutical formulation for administering the decitabine.

Also optionally, the kit may comprise a first container containing decitabine in a solid form; a second container containing a cyclodextrin compound in a solid form; and a third container containing an aqueous diluent comprising at least 60% v/v water, wherein adding the diluent to the solid cyclodextrin compound results in the formation of an aqueous solution which is then added to the solid decitabine to produce a pharmaceutical formulation for administering the decitabine.

Mixing the solid decitabine and diluent may optionally form a pharmaceutical formulation that comprises between 0.1 and 200 mg decitabine per ml of the diluent, optionally between 1 and 100, between 2 mg and 50 mg, 5 mg and 30 mg, between 10 mg and 25 mg per ml of the solvent

The kit may optionally further include instructions. The instructions may describe how the solid compound(s) and the diluent should be mixed to form a pharmaceutical formulation. The instructions may also describe how to administer the resulting pharmaceutical formulation to a patient. It is noted that the instructions may optionally describe the administration methods according to the present invention.

The diluent and decitabine maybe contained in separate vessels. The vessels may come in different sizes. For example, the vessel may comprise between 1 and 50, 1 and 25, 1 and 20, or 1 and 10 ml of the diluent.

Yet another aspect of the invention relates to use of a pharmaceutical composition in the preparation of a medicament for treating a disease selected from the group consisting of benign tumors, cancer, hematolopical disorders, atherosclerosis, insults to body tissue due to surgery, abnormal wound healing, abnormal angiogenesis, diseases that produce fibrosis of tissue, repetitive motion disorders, disorders of tissues that are not highly vascularized, and proliferative responses associated with organ transplants. The pharmaceutical formulation of the present invention may be for administration orally, parenterally, topically, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously intraadiposally, intraarticularly, or intrathecally. Preferably, the pharmaceutical formulation is for administration intravenously, intramuscularly, or subcutaneously.
Preferably, the pharmaceutical composition is further diluted with an aqueous diluent prior to administration to the patient. Preferably, the aqueous diluent is infusion fluid and the diluted pharmaceutical composition is for intravenous infusion. Preferably, the dilution is performed 10 hr, 2 hr, 1 hr, 30 min, 10 min, 5 min or less before administration to the patient. Preferably, said medicament is for administration with a therapeutic agent other than decitabine.

The therapeutic agent may be selected from the group consisting of anti-neoplastic agents, alkylating agents, agents that are members of the retinoids superfamily, hormonal agents, plant-derived agents, biologic agents, interleukins, interferons, cytokines, immuno-modulating agents, and monoclonal antibodies.

Related to the kit, a method is also provided that comprises mixing a decitabine and a cyclodextrin compound that are in a solid, preferably powder form with an aqueous diluent comprising at least 60% v/v water to form a pharmaceutical composition.

In another embodiment, the method comprises : mixing decitabine that is in a solid preferably powder form with an aqueous diluent comprising a cyclodextrin compound solvated in at least 60% v/v water to form a pharmaceutical formulation.

Advantageously, the pharmaceutical formulation may be formed by mixing the decitabine with the diluent shortly prior to administration to a patient (e.g., within one day, or even 6, 5, 4, 3, 2 or 1 hours or less before administration). This reduces decomposition of the cytidine analog/derivative Optionally, as described herein, the pharmaceutical formulation is suitable for administration by admixing aliquots of the pharmaceutical formulation with an aqueous solution (e.g., infusion fluid); and infusing the resulting solution into the patient's body, optionally with a Y connector as also described herein.

BRIEF DESCRIPTION OF THE FIGURES

**Figure 1** shows results of a study on the stability of decitabine formulated with cyclodextrins at 2-8°C.

**Figure 2** shows results of a study on the stability of decitabine formulated with cyclodextrins at 25°C.

**Figure 3** shows results of a study on the stability of decitabine in solutions with various HPBCD concentrations at 25°C.

**Figure 4** shows UV spectra of decitabine in cyclodextrin solutions at 25°C.

**Figure 5** shows UV spectra of decitabine in solutions with various HPBCD concentrations at 25°C,

**Figure 6** is a plot of Delta A of 262 nm against 1/[HPBCD].

DETAILED DESCRIPTION OF THE PRESENT INVENTION

The present invention provides improved pharmaceutical formulations of cytidine analogs, e.g., decitabine which can be used for the treatment of various diseases and conditions, such as myelodysplastic syndrome (MDS), non-small cell lung (NSCL) cancer, and sickle-cell anemia. This innovative approach is taken to overcome three major hurdles that have adversely impacted the commercial development of this type of drugs: hydrolytic degradation in aqueous environment; low solubility in most pharmaceutically acceptable solvents; and minimal oral bioavailability.

As discussed above, in the current clinical formulation decitabine is only slightly soluble in aqueous solutions such as normal saline, and undergoes rapid degradation once dissolved. The instability and limited solubility of decitabine in aqueous solution creates serious inconvenience in both manufacturing and clinical use of decitabine drug product. The instability of dectabine in aqueous solution requires refrigeration of decitabine solution fluting manufacturing cold infusion fluids for drug dilution before intravenous administration, and short storage time of decitabine aqueous solutions. The limited solubility in aqueous solution also requires large volume of the drug to be administered, which makes it difficult to administer the drug subcutaneously.

The present invention provides innovative, solutions to the above problems associated with the current clinical formulation of decitabine. The inventors discovered that cyclodextrin compounds can significantly increase the solubility decitabine in aqueous solutions, and to dramatically improve its stability in the aqueous solutions. The inventors believe that this invention would minimize or eliminate much of the serious inconvenience in manufacturing, storage and transportation of the decitabine drug product. More importantly, in clinical applications of a drug product of decitabine, the present invention can reduce patient disconfort and inconvenience by eliminating the need for cold infusion, and lower the volume of the drug solution to be administered, especially for subcutaneous administration

I. Cyclodextrin in the Formulations of the Present Invention

Accordingly to the present invention, the cyclodextrin compound may be an α, β-, or γ-cyclodextrin Cyclodextrins are cyclic oligomers of glucose; these Compounds form inclusion complexes with another compound whose molecule can fit into the lipopophile seeking cavities of the cyclodextrin molecule. Specifically, α-cyclodextrin contains six glucopyranose units; β-cyclodextrin contains seven glucopyranose units; and γ-cyclodextrin contains eight glucopyranose units. Typically, a cyclodextrin molecule is believed to form a truncated cone having a core opening of 4.7-5.3 Å, 6.0-6.5 Å and 7.5-8.3 Å in α-, β-, or γ-cyclodextrin respectively.

The cyclodextrin compound may be crystalline and amorphous. Preferably the cyclodextrin compound is amorphous. In general amorphous cyclodextrin is prepared by non-selective additions, especially alkylation of the desired cyclodextrin species, Reactions are carried out to yield mixtures containing a plurality of components thereby preventing crystallization of the cyclodextrin. Various alkylated and hydroxyalkyl-cyclodextrins can be made and of course will vary, depending upon the starting species of cyclodextrins and the addition agent used Among the amorphous cyclodextrins suitable for compositions according to the invention are hydroxypropyl, hydroxyethyl, glucosyl, maltosyl and maltotriosyl derivatives of β-cyclodextrin, carboxyamidomethyl-β cyclodextrin, carboxymethyl-β-cyclodextrin, hydroxypropyl-β-cyclodextrin and diethylamino-β-cyclodextrin.

In one embodiment, the cyclodextrin compound is a sulfoalkyether cyclodextrin derivative, preferably mono-tetra or hepta-substitued β-cyclodextrin sulfobutyl ether sodium salt and more preferably β-cyclodextrin sulfobutyl ether, 7 sodium salt (CAPTISOL®, Cydex, Inc, Lenexa, KS).

In another embodiment the cyclodextrin compound is a substituted hydroxy-β-cyclodextrin, preferably hydroxypropyl, hydroxyethyl, glucosyl, maltosyl and maltotriosyl derivatives of β-cyclodextrin.

The cyclodextrin compound may be en unmodified or modified α-, β-, or γ- cyclodextrin. However, unmodified or modified α-, β-, or γ- cyclodextrin is less preferred in the compositions according to the invention because the unmodified forms tend to crystallize and are relatively less soluble in aqueous solutions. More preferred for the compositions according to the invention are the α-, β-, or γ-cyclodextrins that are chemically modified or substituted. Chemical substitution at the 2,3 and 6 hydroxyl groups of the glucoyranose units of the cyclodextrin rings yields increases in solubility of the cyclodextrin compound

Most preferred cyclodextrins in the compositions according the invention are amorphous cyclodextrin compounds. By amorphous cyclodextrin is meant non-crystalline mixtures of cyclodextrins wherein the mixture is prepared from α-, β-, or γ- cyclodextrin. In general, the amorphous cyclodextrin is prepared by non-selective alkylation of the desired cyclodextrin species. Suitable alkylation agents for this purpose include but are not limited to propylene oxide, glycidol, iodoacetamide, chloroacetate, and 2- diethylaminoethlychloride. Reactions are carried out to yield mixtures containing a plurality of components thereby preventing crystallization of the cyclodextrin. Various alkylated cyclodextrins can be made and of course will vary, depending upon the starting species of cyclodextrin and the alkylating agent used. Among the amorphous cyclodextrins suitable for compositions according to the invention are hydroxypropyl, hydroxyethyl, glucosyl, maltosyl and maltotriosyl derivatives of β-cyclodextrin, carboxyamidomethyl-β-cyclodaxtrin, carboxymethyl-β-cyclodextrin, hydroxypropyl-β-cyclodaxtrin and diethylamino-β-cyclodextrin.

In the compositions according to the invention hydroxypropyl-β-cyclodextrin is preferred although the α-or γ-analogs may also be suitable. The particular alkylated α-, β-, or γ- cyclodextrin to be used with the particular cytidine analog or derivative to form the compositions according to the invention will be selected based on the size of the molecule of the cytidine analog or derivative and the relative size of the cavity of the cyclodextrin compound. As with the unsubstituted cyclodextrins mentioned above, it may be advantageous to use alkylated cyclodextrin having a larger cavity when the composition according to the invention also includes an excipient. The use of a particular α-, β-, or γ-cyclodextrin with a particular cytidine analog/derivative, or cytidine analog/derivative and excipient in the compositions according to the invention may of course be optimized based on the effectiveness in of maintaining the particular cytidine analog/derivative in solution.

The composition according to the invention may comprise a mixture of two or more of α-, β-, or γ-cyclodextrin. Preferably, the composition according to the invention will comprise only one of the α-, β-, or γ-cyclodextrins.


## 2. Preparation of Pharmaceutical Formulations

The compositions of the present invention are suitable for administration by any route, preferably in the form of a pharmaceutical composition adapted to such a route, as illustrated below and are dependent on the condition being treated. The formulations are, for example, suitable for administration orally, parenterally, intraperitoneally, intravenously, intraarterially, transdermally, sublingually, intramuscularly, rectally, transbuccally, intranasally, liposomally, via inhalation, vaginally, intraoccularly, via local delivery (for example by a catheter or stent), subcutaneously, intraadiposally, intraarticularly, or intrathecally.

For oral administration, the compositions can be in the form of, for example, a capsule, suspension or liquid. The pharmaceutical composition is preferably made in the form of a dosage unit containing a therapeutically-effective amount of the active ingredient.

Alternatively, the compositions of the present invention can be in powder form, or preferably lyophilized powder form, for reconstitution in the appropriate pharmaceutically acceptable carrier at the time of delivery.

The pharmaceutical compositions are suitable for administration via injection. Formulations for parenteral administration can be in the form of aqueous isotonic sterile injection solutions or suspensions. As described above, these solutions or suspensions can be prepared from sterile powders or granules having decitabine and also including one or more of the cyclodextrin compound. The powders or granules can be dissolved in water, aqueous buffer or a solvent comprising at least 60% v/v water.

In a preferred embodiment, the decitabine can be formulated into a pharmaceutically acceptable composition comprising the decitabine solvated in an aqueous solution. It is believed that the solubility and/or stability of the decitabine will be improved in such pharmaceutical formulations so that the pharmaceutical formulations may be stored for a prolonged period of time prior to use or require less volume of liquid for injection.

As discussed above, in current clinical treatment with decitabine, to minimize drug decomposition decitabine is supplied as lyophilized powder (together with $KH_2PO_4$ and NaOH) which is reconstituted with sterile water for injection, and diluted in cold infusion fluids prior to administration. Such a formulation and treatment regimen suffers from a few drawbacks. First, refrigeration of decitabine in cold solution becomes essential, which is burdensome in handling and economically less desirable than a formulation that can sustain storage at higher temperatures. Second, due to rapid decomposition of decitabine in aqueous solution, the reconstituted and diluted decitabine solution may only be infused to a patient for a maximum of 3 hr if the solution has been stored in the refrigerator for less than 7 hr. In addition, infusion of cold fluid can cause great discomfort and pain to the patient, which induces the patient's resistance to such a regimen.

By forming a complex between the decitabine and the cyclodextrin compound in the solution, the pharmaceutical formulations can circumvent the above-listed problems associated with the current clinical treatment with decitabine. The decitabine can be formulated in aqueous solutions containing water in at least 60% vol. of the solvent, optionally at least 80%, or optionally at least 90% vol. of the solvent. These inventive formulations are believed to be more chemically stable than the current clinical formulation.

The relative amounts of the decitabine and cyclodextrin compound will vary depending upon the relative amount of each of the decitabine and the effect of the cyclodextrin compound on the decitabine. In general, the ratio of the weight of the decitabine to the weight of cyclodextrin compound may be in a range between 1:1 and 1:5000. Within this range, the solubility and/or stability of the decitabine will be significantly increased when the ratio of the weight of decitabine to the weight of cyclodextrin compound is in a range between the concentration at which the decitabine will not go into solution at the particular amorphous cyclodextrin concentration and 1:2000. A weight to weight ratio in a range of 1:1 to 1:200 and more preferably in a range of 1:5 to 1:50 of decitabine to cyclodextrin compound are believed to be the most effective for increased solubility and/or stability of the decitabine. For example, decitabine dissolved in an aqueous solution of cyclodextrin compound in a ratio of between 1:10 and 1:300 (drug:cyclodextrin, wt:wt), and a final concentration of the injection solution of 40 mg/ml of decitabine is expected to significantly increase stability as compared to decitabine in normal saline.

Preferably, if the aqueous solution comprising the decitabine and the cyclodextrin compound is to be administered parenterally, especially via the i.v. route, the cyclodextrin compound will be substantially free of pyrogenic contaminants. Cyclodextrin compound, preferably amorphous hydroxypropyl-β-cyclodextrin may be purchased from a number of vendors including Janssen Pharmaceuticals under the tradename Encapsin. In addition, other forms of amorphous cyclodextrin having different degrees of substitution or glucose residue number are available commercially. A method for the production of hydroxypropyl-β-cyclodextrin is disclosed in Pitha et aL, U.S. Pat. No.4, 727,064 which is incorporated herein by reference.

To produce the formulations according to the invention, a pre-weighed amount of hydroxypropyl-β-cyclodextrin compound, which is substantially pyrogen free is placed in a suitable depyrogenated sterile container. Methods for depyrogenation of containers and closure components are well known to those skilled in the art and are fully described in the United States Pharmacopeia 23 United States Pharmacopeial Convention, Rockville, Md. USA). Generally, depyrogenation is accomplished by exposing the objects to be depyrogenated to temperatures above 250°C for a period of time sufficient to fully incinerate any organic matter. As measured in U.S.P. Bacterial Endotoxin Units, the formulation will contain no more than 10 Bacterial Endotoxin Units per gram of amorphous cyclodextrin. By substantially pyrogen free is meant that the hydroxypropyl-β-cyclodextrin contains less than 10 U.S.P. bacterial codotoxin units per gram using the U.S.P. method. Preferably, the hydroxypropyl-β-cyclodextrin will contain between 0.1 and 5 U.S.P. bacterial endotoxin units per mg, under conditions specified in the United States Pharmacopeia 23.

Sufficient sterile water or aqueous buffer is added to the substantially pyrogen free amorphous cyclodextrin until the desired concentration of hydroxypropyl-β-cyclodextrin is in solution. To this solution a pre-weighed amount of decitabine is added whth agitation and with additional standing if necessary until it dissolves.

The solution may then be filtered through a sterile 0.2 micron filter into a sterile helding vessel and is subsequently filled in sterile depyrogenated vials, optionally lyophilized, and capped. For products that can be stored for long periods of time, a pharmaceutically acceptable preservative may be added to the solution of cytidine analog/derivative and hydroxypropyl-β-cyclodextrin prior to filtration, filling, optionally lyophillization, and capping or alternatively, may be added sterilely after filtration.

Owing to the enhanced stability, the inventive formulation may be stored and transported at ambient temperature, thereby significantly reducing the cost of handling the drug. Further, the inventive formulation may be conveniently stored for a long time before being administered to the patient. In addition, the inventive formulation may be diluted with regular infusion fluid (without chilling) and administered to a patient at room temperature, thereby avoiding causing patients' discomfort associated with infusion of cold fluid.

The decitabine may be dissolved in a solution of cyclodextrin compound at different concentrations. For example, the formulation may optionally comprise between 0.1 and 200; between 1 and 100; between 1 and 50; between 2 and 50; between 2 and 100; between 5 and 100; between 10 and 100 or between 20 and 100 mg decitabine per ml of the solution. Specific examples of the decitabine per solution concentrations include but are not limited to 2, 5, 10, 20, 27, 25, 30, 40 and 50 mg/ml.

The pharmaceutical formulation may further comprise an acidifying agent added to the formation in a proportion such that the formulation has a resulting pH between about 4 and 8. The acidifying agent may be an organic acid. Examples of organic acid include, but are not limited to, ascorbic acid, citric acid, tartaric acid, lactic acid, oxalic acid, formic acid, benzene sulphonic acid, benzoic acid, maleic acid, glatamic acid, succinic acid, aspartic acid, diatrizoic acid, and acetic acid. The acidifyring agent may also be an inorganic acid, such as hydrochloric acid, sulphuric acid, phosphotic acid, and nitric acid.

It is believed that adding an acidifying agent to the formulation to maintain a relatively neutral pH (e.g., within pH 4-8) facilitates ready dissolution of decitabine in the solvent and enhances long-term stability of the formulation. In alkaline solution, there is a rapid reversible decomposition of decitabine to N-(formylamidino)-N'-β-D-2-deoxybofunossylurea, which decomposes irreversibly to form 1-β-D-2'-deoxyribofuranasyl-3-guanylurea. The first stage of the hydrolytic degradation involves the formation of N-amidinium-N'-(2-deoxy-β-D-erythropentofuranosyl)area formate (AUF). The second phase of the degradation at an elevated temperature involves formation of guanidine. In acidic solution, N-(formylamidino) N'-β-D-2-deoxyribofuranesylurea and some unidentified compounds are formed. In strongly acidic solution (at pH <2.2) 5-azacytosine is produced. This maintaining a relative neutral pH may be advantageous for the formulation comprising the decitabine.

In a variation, the acidifying agent is ascorbic acid at a concentration of 0.01-0.2 mg/ml of the solvent, optionally 0.04-0.1 mg/ml or 0.03-0.07 mg/ml of the solvent.

The pH of the pharmaceutical formulation may be adjusted to be between pH 4 and pH 8, preferably between pH 5 and pH 7, and more preferably between pH 5.5 and pH 6.8.

The pharmaceutical formulation is preferably at least 80%, 90%, 95% or more stable upon storage at 2-8°C for 5, 10, 15, 24 hours, or 2, 4, 7, 14, 21, 28 or more days. The pharmaceutical formulation is also preferably at least 88%, 90%, 95% or more stable upon storage at 20-25°C for 5,10, 15, 24 hours, or 2,5,7,14,21,28 or more days.

Optionally, steps may be taken to increase the rate at which the decitabine is solvated by the solution containing a cyclodextrin compound. Examples of additional steps that may be performed include, but are nor limited to, agitation heating, extension of solvation period, and application of micronized cytidine analog/derivative and the combinations thereof.

In one variation, agitation is applied Examples of agitation include but are nor limited to, mechanical agitation, sonication, conventional mixing, conventional stirring and the combinations thereof. For example, mechanical agitation of the formulations may be performed according to manufacturer's protocols by Silverson homogenizer manufactured by Silverson Machines Inc., (East Longmeadow, MA).

In another variation, heat may be applied Optionally, the formulations may be heated in a water bath Preferably, the temperature of the heated formulations may be less than 70°C, more preferably, between 25°C and 40°C. As an example, the formulation may be heated to 37°C.

In yet another variation, a micronized form of the decitabine may also be employed to enhance solvation kinetics. Optionally, micronization may be performed by a milling process. As an example, micronization may be performed according to manufacturer's protocols by jet milling process performed by Malvem Mastersizer, Mastersizerusing an Air Jet Mill, manufactured by Micron Technology Inc. (Boise, ID). IncFluid Energy Aljet Inc. (Boise, ID Telford, PA).

Optionally, the pH of the pharmaceutical formulations may be adjusted by commonly used methods. In one variation, pH is adjusted by addition of acid, such as ascorbic acid, or base, such as sodium hydroxide. In another variation, pH is adjusted and stabilized by addition of buffered solutions, such as solution of (Ethylenedinitrilo) tetraacetic acid disodium salt (EDTA). As decitabine is known to be pH-sensitive, adjusting the pH of the pharmaceutical formulations to approximately pH 7 may increase the stability of therapeutic component.

Optionally, separation of non-dissolved decitabine may be performed for the pharmaceutical formulations. Separation may be perfomed by any suitable technique. For example, a suitable separation method may include one or more of filtration, sedimentation, and centrifugation of the pharmaceutical formulations. Clogging that may be caused by non-dissolved particles of the cytidine analog/derivative, may become an obstacle for administration of the pharmaceutical formulations and a potential hazard for the patient The separation of non-dissolved decitabine from the pharmaceutical formulations may facilitate administration and enhance safety of the therapeutic product.

Optionally, sterilization of the pharmaceutical formulations may be performed. Sterilization may be performed by any suitable technique. For example, a suitable sterilization method may include one or more of sterile filtration, chemical, irradiation, heat filtration, and addition of a chemical disinfectant to the pharmaceutical formulation.

The pharmaceutical formulation may optionally further include an excipient added in an amount sufficient to enhance the stability of the compposition,maintainthe product in solution, or prevent side effects (e.g., potential ulceration, vascular irritation or extravasation) associated with the administration of the inventive formulation. Examples of excipients include, but are not limited to, mannitol, sorbitol, lactose, and dextrose.

Optionnally, the decitabine may be formulated in an oral dosage form. The formulation is preferably a pharmaceutical composition adapted for oral administration, comprising decitabine and a cyclodextrin compound.

Also optionally, the decitabine may be formulated in a topical dosage form. The formulation is preferably a pharmaceutical composition adapted for topical administration, comprising: decitabine and a cyclodextrin compound.

For topical use the compositions of the present invention can also be prepared in suitable forms to be applied to the skin, or mucus membranes of the nose and throat, and can take the form of creams, ointments, liquid sprays or inhalants, lozenges, or throat paints. Such topical formulations further can include chemical compounds such as dimethylsulfoxide (DMSO) to facilitate surface penetration of the active ingredient.

Also optionally, the decitabine may be formulated in a dosage form suitable for application to the eyes or ears. The decitabine or cyclodextin compound can be presented in liquid or semiliquid form formulated in hydrophobic or hydrophilic bases as ointments, creams, lotions, paints or powders.

Also optionally, the decitabine may be formulated in a dosage form suitable for administration through inhalation. The decitabine or cyclodextrin can be present in liquid sprays or mixed with inhalants, lozenges, or throat paints.

Alternatively, the decitabine or the cyclodextrin compound can be in powder form for reconstitution in the appropriate pharmaceutically acceptable carrier at the time or delivery.

When the decitabine or the cyclodextrin is in solid form. Upon dissolving in liquid, Preferably aqueous solution, the decitabine forms complex with the cyclodextrin in the composition. The composition may further comprise buffer salt which may stabilize the cyclodextrin complex.

### 3. Vessels or Kits

The decitabine and/or the cyclodextrin compound described in this invention may be contained in a sterilized vessel such as syringe bottles, and glass vials or ampoules of various sizes and capacities. The sterilized vessel may optionally contain the decitabine and/or the cyclodextrin compound in a form of powder or crystalline, or its solution formulation with a volume of e.g., 1-50 ml, 1-25 ml, 1-20 ml or 1-10 ml. Sterilized vessels enable maintain sterility of the pharmaceutical formulations, facilitate transportation and storage, and allow administration of the pharmaceutical formulation without prior sterilization step.

The present invention also provides a kit for administering decitabine to a host in need thereof, comprising: decitabine in a solid form, and an aqueous diluent that comprises a cyclodextrin compound and at least 60% v/v water. In one embodiment, the kit comprises decitabine and the cyclodextrin compound in a solid, preferably powder form, and an aqueous diluent that comprises water, buffer, excipient or combinations thereof. Mixing of the solid compounds and the diluent preferably results in the formation of a pharmaceutical formulation according to the present invention. For examples, the kit may comprise a first vessel comprising the decitabine, the cyclodextrin compound, and optionally buffer salt (e.g, potassium phosphate), in a solid form; and a vessel container comprising a diluent that comprises water; wherein adding the diluent to the solid compounds results in the formation of a pharmaceutical formulation. Mixing the solid compounds and diluent may optionally form a pharmaceutical formulation that comprises between 0.1 and 200 mg of the cytidine analog/derivative per ml of the diluent, optionally between 0.1 and 100, between 2 mg and 50 mg, 5 mg and 30 mg, between 10 mg and 25 mg per ml of the solvent.

In one embodiment, the diluent is injection or infusion fluid. Examples of injection or infusion fluid include, but are not limited to, BWFI (Racteriostetic Water For Injection), SWFI (Sterile Water For Injection), D5W (Dextrose 5% in Water), D10W (Dextrose 10% in Water), D5LR(Dextrose in Lactate Ringer's Solution D5½S (Dextrose 5% in 1/4 Strength Saline (5% Dextrose and 0.22% Sodium Chloride Injection)), D5½S (Dextrose 5% in 1/2 Strength Saline (5% Dextrose and 0.45% Sodium Chloride Injection)), D5NS (Dextrose 5% in Normal Salins (5% Dextrose and 0.9% Sodium Chloride Injection)). D5R (Dextrose 5% in Ringer's Injection), D10NS (Dextrose 10% in Normal Saline (10% Dextrose and 0.9% Sodium. Chloride Injection)). IS10W (Invert Sugar 10% in Saline (10% Invert Sugar in 0.9% Sodium Chloride Injection)), I.R (Lactated-Ringer's Injection), Pr (protein Hydrolysate Injection), R (Ringer's Injection). NS Sodium Chloride 0.9% (Normal Saline), SOD CL 5 (Sodium Chloride 5% (5% Sodium Chloride Injection), and Sod Lac (Sodium Lactate, 1/6 Molar(M/6 Sodium Lactate Injection)).

In another embodiment, the kit comprises the decitabine in a solid, preferably powder form and a liquid diluent that comprises a cyclodextrin compound dissolved in an aqueous solvent which may comprise water, buffer, excipient or combinations thereof. Mixing of the solid compound and the diluent preferably results in the formation or pharmaceutical formulation according to the present invention. For example, the kit may comprise a first vessel comprising the decitabine in a solid form; and a vessel container comprising the liquid diluent; wherein adding the diluent to the solid compound results in the formation of a pharmaceutical formulation, Mixing the solid compounds and diluent may optionnally form a pharmaceutical formulation that comprises between 0.1 and 200 mg of the cytidine analog/derivative per ml of the diluent optionally between 0.1 and 100, between 2 mg and 50 mg, 5 mg and 30 mg, between 10 mg and 25 mg per ml of the solvent.

The kit may further comprise one or more syringes and/or syringe needles for injection the pharmaceutical formulation to the patient. For the viscous liquid formulation, syringe needles with high fluidic capacity are preferred, such as DEPOTONE syringe needles (Imprint Pharmaceuticals Ltd., UK).

The kit may optionally further include instructions. The instructions may describe how the solid compounds and the diluent should be mixed to form a pharmaceutical formulation The instruction may also describe how to administer the resulting pharmaceutical formulation to a patient. It is noted that the instructions may optionally describe the administration methods according to the present invention.

The diluent and the decitabine may be contained in separated vessels. The vessels may come in different sizes. For example, the vessel may comprise between 1 and 50, 1 and 25, 1 and 20. or 1 and 10 ml of the diluent.

The pharmaceutical formulations provided in vessels or kits may be in a form that is suitable for direct administration or may be in a concentrated form that requires dilution relative to what is administered to the patient. For example, pharmaceutical formulations, described in this invention, may be in a form that is suitable for direct administration via infusion.

The methods and kits described herein provide flexibility wherein stability and therapeutic effect of the pharmaceutical formulations comprising the inventive compound may be further enhanced or complemented

## 4. Methods of Administration

The decitabine formulated with a cyclodextrin compound are suitable for administration by any route, preferably in the form of a pharmaceutical composition adopted to such a route, as illustrated below and are dependent on the condition being treated. The compounds or formulations are, for example, suitable for administration orally, parenterally, topically intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly, or intrathecally. The compounds and/or compositions according to the invention are also suitable for administration or co-administration in slow release dosage forms.

The decitabine formulated with a cyclodextrin compound may be for administration or co-administration in any conventional dosage form, Co-administration in the context of this invention is defined to mean the administration af more than one therapeutic agent in the course of a coordinated treatment to achieve an improved clinical outcome. Such co-administration may also be coextensive, that is, occuring during overlapping periods of time.

The decitabine may be administered into a host such as a patient at a dose of 0.1-1000 mg/m$^2$, optionally 1-200 mg/m$^2$, optionally 1-150 mg/m$^2$ optionally 1-130 mg/m$^2$, optionally 1-75 mg/m$^2$, optionally 1-50 mg/m, optionally1-40mg/m, optionally 1-30 mg/m$^2$, optionally 1-20 mg/m$^2$, or optionally 5-30 mg/m$^2$.

For example, decitabine and the cyclodextrin compound may be supplied as sterile powder for injection, optionally together with buffering salt such as potassium dihydrogen phosphate and pH modifier such as sodium hydroxide. This formulation is preferably stored at 2-25°C, which should keep the drug stable for at least 3 years. This powder formulation may be reconstituted with 10 ml of sterile water for injection-This solution may be further-diluted with infusion fluid known in the art, such as 0.9% sodium chloride injection, 5% dextrose injection and lactated ringer's injection, preferably by employing a Y-connector. It is preferred that the reconstituted and diluted solutions be used within 10-11 hours for delivery of maximum potency.

In a preferred embodiment the decitabine formulated with a cyclodextrin compound is suitable for administration to a patient by injection, such as subcutaneous injection, bolus i.v. injection, continuous i.v. infusion and i.v. infusion over 1 hour. Optionally the inventive compound/composition is for administration to a patient via an 1-24 hour i.v. infusion per day for 3-5 days per treatment cycle at a dose of 0.1-1000 mg/m$^2$ per day, optionally at a dose of 1-200 mg/m$^2$ per day, optionally at a dose of 1-150 mg/m$^2$ per day, optionally at a dose of 1-100 mg/m$^2$ per day, optionally at a dose of 2-50 mg/m$^2$ per day, optionally at a dose of 10-30 mg/m$^2$ per day, or optionally at a dose of 5-20 mg/m$^2$ per day,

For decitabine, the dosage below 50 mg/m$^2$ is considered to be much lower than that used in conventional chemotherapy for cancer. By using such a low dose of the analog/derivative of decitabine transcriptional activity of genes silenced in the cancer cells by aberrant methylation can be activated to trigger downstream signal transduction, leading to cell growth arrest, differentiation and apoptosis, which eventually results in death of these cancer cells. This low dosage, however, should have less systemic cytotoxic effect on normal cells, and thus have fewer side effects on the patient being treated

The decitabine formulated with a cyclodextrin compound may be co-administered in any conventional form with one or more member selected from the group comprising infusion fluids, therapeutic compounds, nutritious fluids, anti-microbial fluids, buffering and stabilizing agents.

As described above, the decitabine can be formulated with a cyclodextrin compound in a liquid form by solvating the drug and cyclodextrin compound in an aqueous solvent. The pharmaceutical liquid formulations provide the further advantage of being directly administrable, i.e., without further dilution, and thus can be stored in a stable form until administration. Further, because the formulation can be readily mixed with water or infusion/injection fluid, the formulations can be easily and readily further diluted just prior to administration. For example, the pharmaceutical formulations can be diluted with water or infusion/injection fluid 180, 60, 40, 30, 20, 10, 5, 2, 1 minute or less before administration to a patient

Patients may receive the pharmaceutical formulations parenterally. The preferred route of administration is by intravenous infusion or injection, or by subcutaneous injection. Optionally, the pharmaceutical formulations of the current invention may be infused directly, without prior dilution.

In one embodiment, the pharmaceutical formulation comprising the decitabine and cyclodextrin compound is infused through a connector, such as a Y site connector, that has three arms, each connected to a tube. As an example, Baxter® Y-connectors of various sizes can be used. A vessel containing the pharmaceutical formulation is attached to a tube further attached to one arm of the connector. Infusion fluids, such as 0.9% sodium chloride, or 5% dextrose, or 5% glucose, or Lactated Ringer's, are infused through a tube attached to the other arm of the Y-site connector. The infusion fluids and the pharmaceutical formulations are mixed inside the Y site connector. The resulting mixture is infused into the patient through a tube connected to the third arm of the Y site connector. The advantage of this administration approach over the prior art is that the cytidine analog/derivative is mixed with infusion fluids before it: enters the patient's body, thus reducing the time when decomposition of the cytidine analog/derivative may occur due to contact with water. For example, the cytidine analog/derivative is mixed less than 10, 5, 2 or 1 minutes before entering the patient's body.

Patients may be infused with the pharmaceutical formulations for 1, 2, 3, 4, 5 or more hours, as a result of the enhanced stability of the formulations. Prolonged periods of infusion enable flexible schedules of administration of therapeutic formulations.

Alternatively or in addition, speed and volume of the infusion can be regulated according to the patient's needs. The regulation of the infusion of the pharmaceutical formulations can be performed according to existing protocols.

The pharmaceutical formulations may be co-infused in any conventional form with one or more member selected from the group comprising infusion fluids, therapeutic compounds, nutritious fluids, anti-microbial fluids, buffering and stabilizing agents. Optionally, therapeutic components including, but are not limited to, anti-neoplastic agents, alkylating agents, agents that are members of the retinoids superfamily, antibiotic agents, hormonal agents, plant-derived agents, biologic agents, interleukins, interferons, cytokines, immuno-modulating agents, and monoclonal antibodies, may be co-infused with the inventive formulations.

Co-infusion in the context of this invention is defined to mean the infusion of more than one therapeutic agents in a course of coordinated treatment to achieve an improved clinical outcome. Such co-infusion may be simultaneous overlapping, or sequential. In one particular example, co-infusion of the pharmaceutical formulations and infusion fluids may be perfomed through Y-type connector.

In humans, decitabine displayed a distribution phase with a half-life or 7 minutes and a terminal half-life on the order of 10-35 minutes as measured by bioassay. The volume of distribution is about 4,6 L/kg. The short plasma half-life is due to rapid inactivation of decitabine by deamination by liver cytidine deaminase. Clearance in humans is high, on the order of 126 mL/min/kg. The mean area under the plasma curve in a total of 5 patients was 408 $\mu$g/h/L with a peak plasma concentration of 2.01 $\mu$M. In patients decitabine concentrations were about 0.4 $\mu$g/ml (2 $\mu$M) when administered at 100 mg/m$^2$ as a 3-hour infusion. During a longer infusion time (up to 40 hours) plasma concentration was about 0.1 to 0.4 $\mu$g/mL. With infusion times of 40-60 hours, at an infusion rate of 1 mg/kg/h, plasma concentrations of 0.43-0.76 $\mu$g/mL were achieved. The steady-stare plasma concentration at an infusion rate of 1 mg/kg/h is estimated to be 0.2-0.5 $\mu$g/mL. The half-life after discontinuing the infusion is 12-20 min. The steady-state plasma concentration of decitabine was estimated to be 0.31-0.39 $\mu$g/mL during a 6-hour infusion of 100 mg/m$^2$. The range of concentrations during a 600-mg/m$^2$ infusion was 0.4-16 $\mu$g/mL. Penetration of decitabine into the cerebrospinal fluid In man reaches 14-21% of the plasma concentration at the end of a 36-hour intravenous infusion. Urinary excretion of unchanged decitabine is low, ranging from less than 0.01% to 0.9% of the total dose, and there is no relationship between excretion and dose or plasma drug levels. High clearance values and a total urinary excretion of less than 1% of the administered dose suggest that decitabine is climinated rapidly and largely by metabolic processes.

Owing to their enhanced stability in comparison with current clinical formulations of decitabine the inventive formulation comprising a cyclodextrin compound can enjoy longer shelf life when stored and circumvent problems associated wich clinical use of decitabine or azactidine. For example, decitabine may be supplied as lyophilezed powder with a cyclodextrin compound, optionally with acid (e.g., ascorbic acid), alkaline (sodium hydroxide), or buffer salt (monobasic potassium dihydrogen phosphate).The lyophilized powder can be reconstituted with sterile water for injection, e.g., i.v., i.p., im, or subcutaneously. Optionally, the powder can be reconstituted with aqueous solvent comprising a water miscible solvent such as glycerin, propylene glycol, ethanol and PEG. The resulting solution may be administered directly to the patient, or diluted further with infusion fluid, such as 0.9% Sodium Chloride; 5% Dextrose; 5% Glucose; and Lactated Ringer's infusion fluid.

The inventive formulations may be stored under ambient conditions or in a controlled environment at 2-25°C (32-77°F). In addition, due to the complex formation and enhanced chemical stability, the inventive formulations may be more resistant to enzymatic deamination and hydrolytic degradation, and should have a longer plasma half-life compared to the current clinical formulation of decitabine.

In addition, due to their enhanced chemical stability, the inventive formulations should have a longer plasma half-life compared to the current clinical formulation of decitabine. Thus, decitabine may be administered to the patient at a lower dose and/or less frequently then that for decitabine. 5. Indications for the Pharmaceutical Formulations and Combination Therapy

The inventive formulations described herein have many therapeutic and prophylactic uses. In a preferred embodiment, the decitabine formulated with a cyclodextrin compound are for use in the treatment of a wide variety of diseases that are sensitive to the treatment with a cytidine analog/derivative, such as decitabine or azacitidine. Preferable indications that may be treated using the inventive formulations include those involving undesirable or uncontrolled cell proliferation. Such indications include benign tumors, various types of cancers such as primary tumors and tumor metastatis, resistenosis (e.g. coronary, carotid, and cerebral lesions), hematological disorders, abnormal stimulation of endothelial cells (atheroselerosis), insults to body tissue due to surgery, abnormal wound healing, abnormal angiogenesis, diseases that produce fibrosis of tissue, repetitive motion disorders, disorders of tissues that are not highly vascularized, and proliferative responses associated with organ transplants.

Generally, cells in a benign tumor retain their differentiated features and do not divide in a completely uncontrolled manner. A benign tumor is usually localized and nonmetastatic. Specific types benign tumors that can be treated using the present invention include hemangiomas, hepatocellular adenoma, cavernous haemangioma, focal modular hyperplasis, acoustic neuromas, neurofibroma, bile duct adenoma, bile duct cystanoma, fibroma, Lipomas, leiomyomas, mesotheliomas, teratomas, myxomas, nodular regenerative hyperplasis, trachomas and pyogenic granulomas.

In a malignant tumor cells become undifferentiated, do not respond to the body's growth control signals, and multiply in an uncontrolled manner. The malignant tumor is invasive and capable of spreading to distant sites (metastasizing). Malignant tumors are generally divided into two categories: primary and secondary. Primary tumors arise directly from the tissue in which they are found. A secondary tumor, or metastasis, is a tumor which is originated elsewhere in the body but has now spread to a distant organ. The common routes for metastasis are direct growth into adjacent structures, spread through the vascular or lymphatic systems, and tracking along tissue planes and body spaces (peritoneal fluid, cerebrospinal fluid, etc.)

Specific types of cancers of malignant tumors, either primary or secondary, that can be treated using this invention include breast cancer, skin cancer, bone cancer, prostate cancer, liver cancer, lung cancer, brain cancer, cancer of the larynx, gall blander, pancreas, rectum, parathyroid, thyroid, adrenal, neural tissue, head and neck, colon, stomach, bronchi, kidney, basal cell carcinoma, squamous cell carcinoma of both ulcerating and papillary type, metastatic skin carcinoma, osteo sarcoma, Ewing's sarcoma, veticulum cell sarcoma, myeloma, giant cell tumor, small-cell lung tumor, gallstones, islet cell tumor, primary brain tumor, acute and chronic lymphocytic and granulocytic tumors, hairy-cell tumor, adenoma, hyperplasia, medullary carcinoma, pheochromocytoma, mucosal neuronms, intestinal gangilonemomas, hyperplastic corneal nerve tumor, marfanoid habitus tumor, Wilm's tumor, seminoma, ovarian tumor, leiomyomater tumor, cervical dysplasia and in situ carcinoma, neuroblastoma, retinoblastoma, soft tissue sarcoma, malignant carcinoid, topical skin lesion, mycosis fungoide, rhabdomyosarcoma, Kaposi's sarcoma, osteogenic and other sarcoma, malignant hypercalcemia, renal cell tumor, polycythermia vera, adenocarcinoma, glioblastoma multiforma, leukemias, lymphomas, malignant melanornas, epidermoid carcinomas, and other carcinomas and sarcomas.

Hematologic disorders include abnormal growth of blood cells which can lead to dysplastic changes in blood cells and hematologic malignancies such as various leukemias. Examples of hematologic disorders include but are not limited to acute myeloid leukemia, acute promyelocytic leukemia, acute lymphoblastic leukemia, chronic myelogenous leukemia, the myelodysplastic syndromes, and sickle cell anemia.

In some embodiments, the inventive formulations are for use in treating blood disorders, including inherited blood disorders and/or disorders where hemoglobin is defective, e.g., sickle cell anemia. In some embodiments, the inventive formulations can be used to treat cancer, including leukemia, pre-leukemia, and other bone narrow-related cancers, e.g., myelodysplatic syndrome (MDS), as well as lung cancer, c.g., non-small cell lung cancer (NSCL) NSCL can include epidermoid or squamous carcinnoma, adenocarcinoma, and large cell carcinoma, MDS can include refractory anemia, refractory anemia with ringed sideroblasts, refractory anemia with excess blasts, refractory anemia with excess blasts in transformation, and chronic myelomonocytic leukemia.

The present invention, provides pharmaceutical compositions, and kits for use in the treatment of animal subjects, The term "animal subject' as used herein includes humans as well as other mammals. The term "treating" as used herein includes achieving a therapeutic benefit and/or a prophylactic benefit. By therapeutic benefit is meant eradication or amelioration of the underlying disorder being treated. For example, in patient with sickle cell anemia, therapeutic benefit includes eradication or amelioration of the underlying sickle cell anemia. Also, a therapeutic benefit is achieved with the eradication or amelioration of one or more of the physiological symptoms associated with the underlying disorder such that an improvement is observed in the patient, notwithstanding the fact that the patient may still be afflicted with the underlying disorder. For example, the inventive formulations provide therapeutic benefit not only when sickle cell anemia is eradicated, but also when an improvement is observed in the patient with respect to other disorders or discomforts that accompany sickle cell anemia, like hand-foot syndrome, fatigue, and or the severity or duration of pain experienced during a crisis (painful episode). Similarly, the inventive formulations can provide therapeutic benefit in ameliorating symptoms associated with cancers, e.g., MDS or NSCr, including anemia, bruising, persistent infections, the size of a lung tumor, and the like.

For prophylactic benefit, the inventive formulations may be administered to a patient at risk of developing a cancer or blood disorder, or to a patient reporting one or more of the physiological symptoms of such a condition, even though a diagnosis of the condition may not have been made.

If necessary or desirable, the inventive formulation may be for administration in combination with other therapeutic agents. The choice of therapeutic agents that can be co-administered with the compounds and compositions of the invention will depend, in part, on the condition being treated,

Examples of the therapeutic agent other than the decitabine include, but are not limited to, alkylating agents, antibiotic agents, antimetabolic agents, hormonal agents, plant-dedved agents, and biologic agents.

Examples of alkylating agents include, but are not limited to, bischloroethylamines (nitrogen mustards, e.g. chlorambucil, cyclophosphamide, ifosfamide, mechlorethamine, melphalan, uracil mustard), aziridines (e.g. thiotepa), alkyl alkone sulfonates (e.g. busafan), nitrosoureas (e.g. cannustine, lomustine, streptozocin.), nonclassic alkylating agents (altretamine, dacarbazine, and procarbazine), platinum compounds (carboplastin and cisplatin).

Examples of antibiotic agents include, but are not limited to, anthracyclines (e.g. doxorubicin, daunorubicin, epirubicin, idarubicin and anthracenedione), mitomycin C, bleomycin, dactinomycin, plicatomycin.

Examples of antimetabolic agents include, but arc not limited to, fluorouracil (5-FU), floxuridine (5-FUdR), methotrexate, leucovorin, hydroxyurea, thioguanine (6-TG), mercaptopurine (6-MP) cytarabine, pentostatin, fludarabine phosphate, cladribine (2-CDA), asparaginase, imatinib mesylate (or GLEEVAC®), and gemcitabine.

Examples of such hormonal agents are synthetic estrogens (e.g. diethylstibestrol), antiestrogens (e.g. tamoxifen, toremifene, fluoxymesterol and raloxifene), antiandrogens (bicalutamide, milutamide, flutamide), aromatase inhibitors (e.g., aminoglutethimide, anastrozole and tetrazole), ketoconazole, goserelin acetate, leuprolide, megestrol acetate and mifepristone.

Examples of plant-derived agents include, but are not limited to, camptothecins (20(S)-camptothecin, 9-nitro-20(S)-camptothecin, 9 amino-20(S)-camptothecin, irotecan, CPT-11), vinca alkaloids (e.g., vincristine, vinblastine, vindesine, vinzolidine and vinorelbine), podophyllotoxins (e.g., etoposide (VP-16) and teniposide (VM-26)), and taxanes (e.g., paclitaxel and docetaxel).

Examples of biologic agents include, but are not limited to, immuno-modulating proteins such as cytokines, monoclonal antibodies against tumor antigens, tumor suppressor genes, and cancer vaccines. Examples of interleukins that may be used in conjunction with CPT include, but are not limited to, interleukin 2 (IL-2), and interleukin 4 (IL-4), interleukin 12 (IL-12). Examples of interferons that may be used in conjunction with CPT include, but are not limited to, interferon a, interferon b (fibroblast interferon) and interferon g (fibroblast interferon)- Examples of such cytokines include, but are not limited to erythropoietin (epoietin a), granulocyte-CSF (filgrastin), and granulocyte, macrophage-CSF (sargramostim)- Other immuno-modulating agents other than cytokines include, but are not limited to bacillus Calmette-Guerin, lovamisole, and octreotide.

Example of monoclonal antibodies against tumor antigens that can be used in conjunction with CPT include, but are not limited to, HERCEPTIN® (Trastuzumab), RITUXAN® (Rituximab), MYLOTARG® (gemtuzumab ozogamicin), CAMPATH® (alemtuzumab), ZEVALIN® (ibritumomab yiuxetan), PANOREX® (edrecolomab), BEXXAR® (tositumomab), ERBITUX® (cetuximab), and AVASTIN® (bevacizumab).

Examples of the tumor suppressor genes include, but arc not limited to, DPC-4, NF-J, NF-2, RB, *p53, WTI,* BRCA1 and *BRCA2.*

Exemple of cancer vaccines include, but are not limited to gangliosides (GM2), prostate specific antigen (PSA), α-fetoprotein (AFP), carcinoembryonic antigen (CEA) (produced by colon cancers and other adenocarcinomas, e.g. breast, lung, gastric, and pancreas cancer s), melanoma associated antigens (MART-1 gp100, MAGE 1,3 tyrosinase), papillomavirus E6 and E7 fragments, whole cells or portions/lysates of antologous tumor cells and allogeneic tumor cells.

An adjuvant may be used to augment the immune response to TAAs. Examples of adjuvants include, but are not limited to, bacillus Calmette-Guerin (BCG), endotoxin lipopolysaccharides, keyhole limpet hemocyamin (GKLH), interleukin-2 (IL-2), granulocyte-macrophage colony-stimulating factor (GM-CSF) and cytoxan, a chemotherapeutic agent which is believe to reduce tumor-induced suppression when given in low doses.

The present invention also relates to a pharmaceutical formulation according to the invention for use in treating undesired or uncontrolled angiogenesis. One or more anti-angiogenesis agents may also be used in combination with the formulation of the invention.

Examples of anti-angiogenesis agents include, but are not limited to, retinoid acid and derivatives thereof 2-methoxyestradiol ANGIOSTATIN™ protein, ENDOSTATIN™ protein, suramin, squalamine, tissue inhibitor of metalloproteinase-I tissue inhibitor of metalloproteinase-2, plasminogen activator inhibitor-1, plasminogen activator inhibitor-2, cartilage-derived inhibitor, paclitaxel, platelet factor 4, protamine sulphate (clupeine), sulphated chitin derivatives (prepared from queen crab shells), sulphated polysaccharide peptidoglycan complex (sp-pg), staurosporine, modulators of matrix metabolism, including for example, proline analogs ((I-azotidine-2-carboxylic acid (LACA.), cishydroxyproline, d,1-3,4-dehydroproline, thiaproline], $\alpha$, $\alpha$-dipyridyl, beta,- aminopropionitrile fumarate, 4-propyl-5-(4-pyridinyl)-2(3h)-oxazolone; methotrexate, mitoxantrone, heparin, interferons, 2 macroglobulin-serum, chimp-3, chymostatin, beta.-cyclodextrin tetradecasulfate, eponemycin; fumagillin, gold sodium thiomalate, d-penicillamine (CDPT), beta.-1-anticollagenase-serum, alpha-2-antiplasmin, bisantrene, lobenzarit disodium, n-(2-carboxyphenyl-4-chloroanthronilic acid disodium or "CCA", thalidomide; angostatic steroid, cargboxynaminolmidazole;metalloproteinase inhibitors such as BB94. Other anti-angiogenesis agents include antibodies, such as monoclonal antibodies against these angiogenic growth factors: bFGF, aFGP, FGF-5, VEGF isoforms, VEGF-C, HGF/SF and Ang-1/Ang-2.

## EXAMPLES

The following examples are intended.to illustrate details of the invention, without thereby limiting it in any manner. As described in the examples below, the use of cyclodextrins as excipients in an aqueous solution can significantly increase the solubility and/or stability of a cytidine analog or derivative such as decitabine and 5-azacytidine.

## 1. Solubility of Decitabine in Cyclodextrin Solutions

Aqueous solutions of cyclodextrin at pH 6.7-7.2 were prepared by dissolving 4 parts of cyclodextrins (hydroxypropyl $\alpha$-cyclodextrin (HPACD), hydroxypropyl $\beta$-cyclodextrin (HPBCD), $\beta$-cyclodextrin (BCD), hydroxypropyl $\gamma$-cyclodextrin (HPBCD), or CAPTISOL) in 6 parts of potassium phosphate buffer (50 mM $KH_2P0_4$, pH 7.0), resulting solutions of 40% w/w cyclodoxtrins. Solid docitabine (SuperGen, Inc., Dublin, CA) was added to the aqueous solution of cyclodextrin and mixed at room temperature (20-25 °C). Table 1 lists the solubility of decitabine in each of the cyclodextrin solutions in comparison with that in the potassium phosphate buffer (pH 7.0).

As shown in **Table 1,** $\beta$-cyclodextrins such as HPBCD and Captisol are more effective in enhancing decitabine solubility in aqueous solutions than $\alpha$-, or $\gamma$-cyclodextrins (such as HPACD and HPGCD). The solubility increase would translate into less volume required per dosage clinically, and would make subcutaneous administration of decitabine drug more practical.

**Table 1**

|  | KPi (pH 7.0) | 40%HPBCD (w/w, pH 7.2) | 40%Captisol (w/w, pH 6.7) | 40%HPGCD (w/w, pH 7.0) | 40%HPACD (w/w, pH 7.0) |
|---|---|---|---|---|---|
| Solubility (mg/mL) | 18 | 44 | 42 | 22 | 18 |

## 2. Stability of Decitabine in Cyclodextrin Solutions

The chemical stability of decitabine in the cyclodextrin solutions and in the phophate buffer as prepared above was determined. The initial decitabine concentration for all the solutions was 17-18 mg/mL at the beginning of the experiment at 2-8°C; 9-10 mg/mL at the beginning of the experiment at 25°C. Decitabine potency was assayed by a HPLC method, and expressed as a percentage of total peak areas at 220 nm.

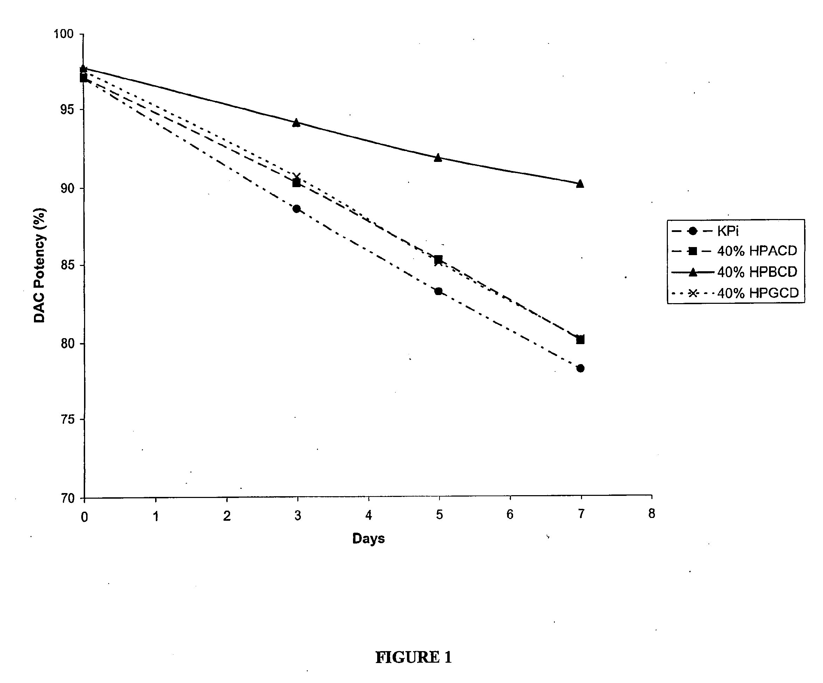

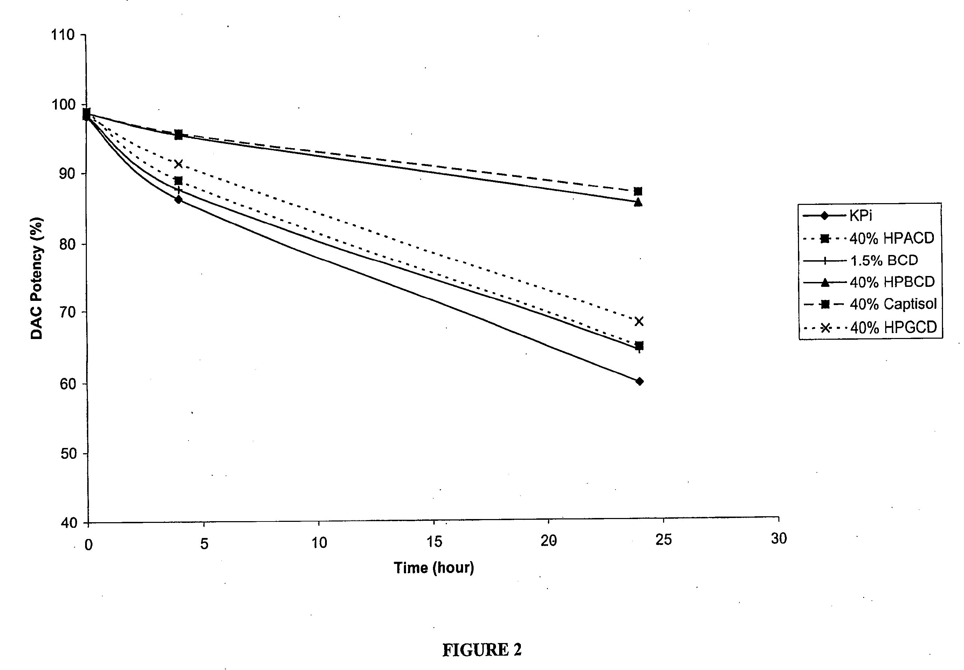

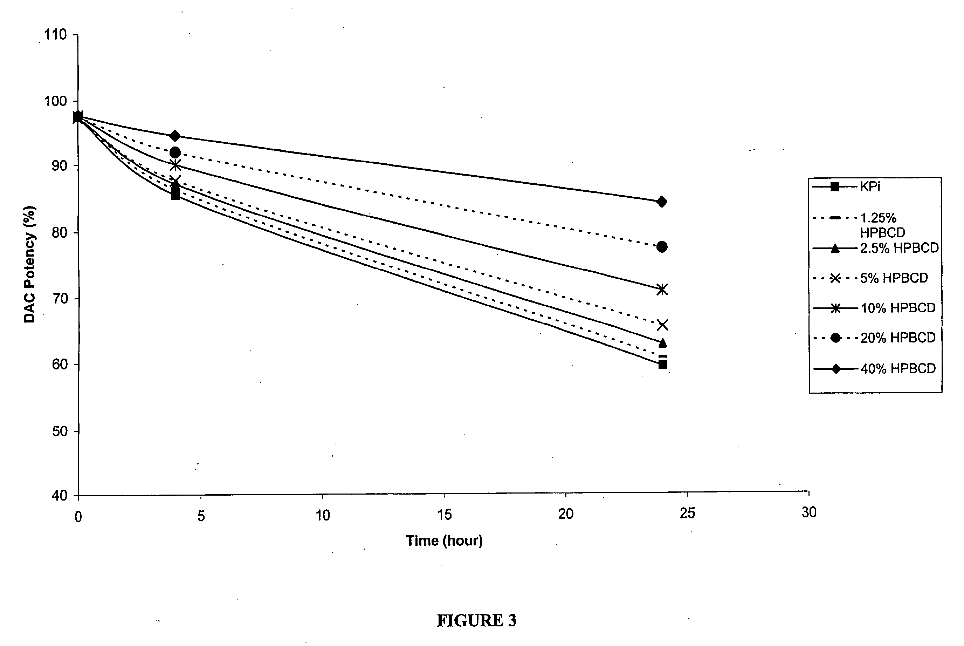

**Figure 1** and 2 shows that the use of cyclodextrins as excipients in an aqueous solution can dramatically improve the stability of decitabine in the solution both at 2-8°C and 25°C, respectively. The stabilization effect of cyclodextrins on decitabine in aqueous solutions is more evident when $\beta$-cyclodextrins such as HPBCD and Captisol are used, compared to $\alpha$-, or $\gamma$-cyclodextrins (HPACD and HPGCD). This stabilization effect is also concentration dependent, as shown in **Figure 3.**

Without being bound to the theory or mechanisms of action, the inventors believe that both the solubility enhancement and stability improvement of decitabine in cyclodextrins-containing aqueous solutions are due to complex formation between decitabine and cyclodextrin molecules, and that $\beta$-cyclodextrins form a tighter, reversible complex with decitabine than $\alpha$-, or $\gamma$-cyclodextrins.

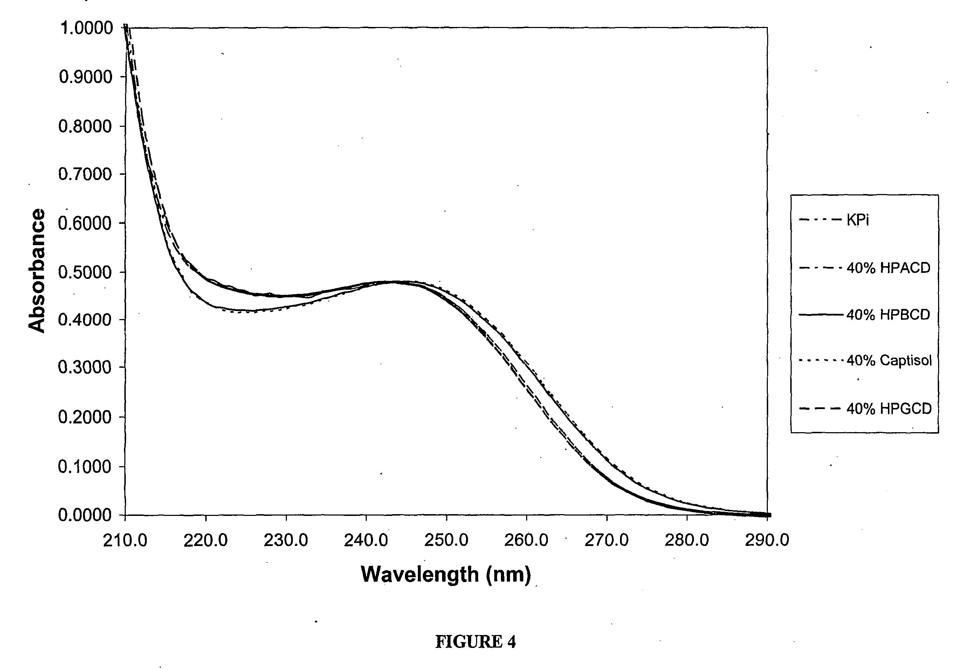

UV spectroscopy study of decitabine in cyclodextrin-containing aqueous solutions was conducted to demonstrate the formation of complex between decitabine and $\beta$-cyclodextrins. Each of the spectra was measured at 0.01-0.02 mg/mL of decitabine at room temperature, after blanked with the same solution devoid of decitabine. All of the spectra are normalized to 69 $\mu$M of decitabine, assuming the extinction coefficient of decitabine at the apex between 243 nm and 246 nm remains the same (7000 $M^{-1}cm^{-l}$). **Figure 4** shows changes of decitabine UV spectrum when $\beta$-cyclodextrins are present **(Figure 4),** strongly suggesting complex formation between decitabine and $\beta$-cyclodextrins.

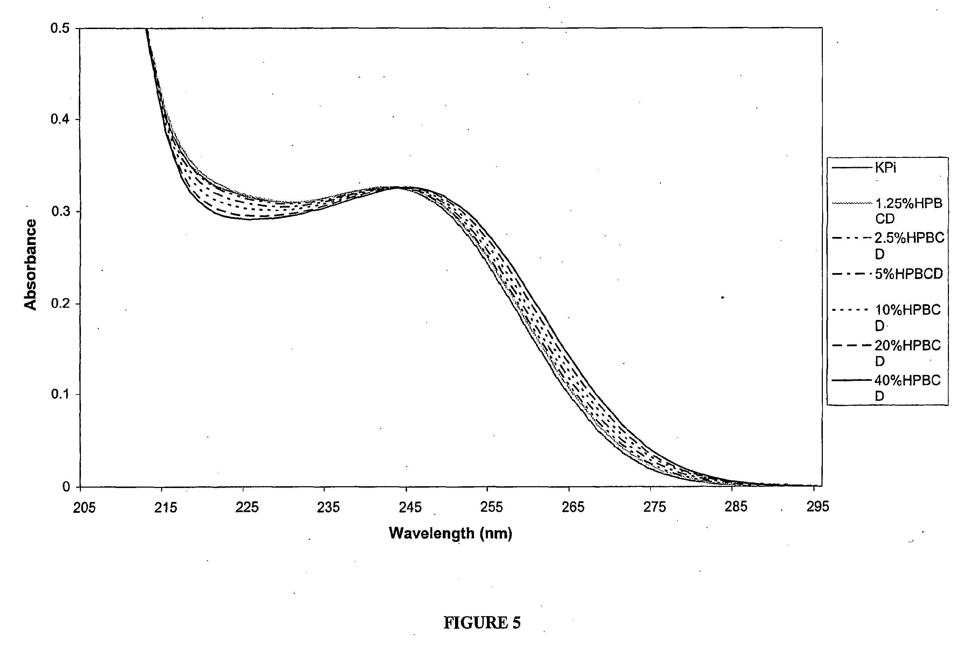

The extent of changes of decitabine UV spectrum of decitabine in cyclodextrin solutions is also shown to be concentration-dependent **(Figure 5).** Each of the spectra was measured at 0.01 mg/mL of decitabine at room temperature, after blanked with the same solution devoid of decitabine. All the spectra are normalized to 47 $\mu$M of decitabine, assuming the extinction coefficient of decitabine at the apex between 243 nm and 246 nm remains the same (7000 M$^{-1}$cm$^{-1}$).). Based on the adsorbance at 262 mn derived from each of the spectra shown in **Figure 5** and the corresponding HPBCD concentration, the complex formation constant K$_f$ for decitabine-HPBCD complex can be calculated based on the following equation (1), assuming the stoichiometry of decitabine/HPBCD complex is 1:1:

$$1/\Delta A = 1/(\Delta A_o * K_f * [\text{HPBCD}]) + 1/\Delta A_o \qquad (1)$$

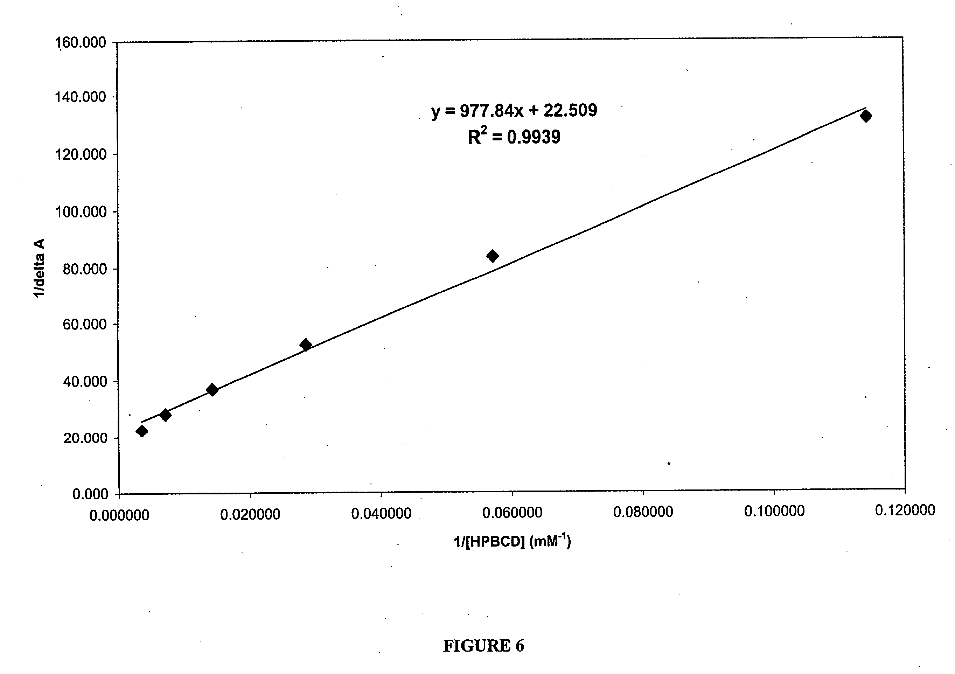

Here, $\Delta A_o$ is the difference in absorbance at 262 nm between the free and the complexed decitabine, and $\Delta A$ is the difference in absorbance at 262 nm between decitabine-HPBCD and decitabine-KPi at the specific [HPBCD] (HPBCD concentration). **Figure 6** shows the plot of 1/$\Delta A$(1/delta A) versus 1/[HPBCD]. In the plot, delta A ($\Delta A$) is the difference in absorbance at 262 nm between decitabine-HPBCD and decitabine-KPi at each specific HPBCD concentration ([HPBCD]). The line is a last-squares fit using equation (1). From the fitting curve, the K$_r$ for decitabine-HPBCD complexation is calculated in be 23 M$^{-1}$.

The effects of $\alpha$-, or $\gamma$-cyclodroxtrins on both the solubility and stability of decitabine are less dramatic, when compared to $\beta$-cyclodextrins such as HPBCD and CAPTISOL, probably due to the formation of less stable complexes between $\alpha$-, or $\gamma$-cyclodextrins and decitabine molecules. The interaction between decitabine and cyclodextrins is believed to be non-covalent and reversible. Under normal clinical drug dosing conditions for decitabine, and based on the calculated K$_f$ (23 M$^{-1}$) for decitabine-HPBCD, it is expected that most of decitabine should dissociate from its complex with HPBCD once infused into patients, and no new drug entity should be formed.

The above results demonstrate that the use of cyclodextrins as excipients in an aqueous solution can increase decitabine solubility in the solution to more than two fold, and also dramatically improve the stability of decitabine in the solution. Cyclodextrins as excipients for decitabine drug can be applied during manufacturing of the drug product, or used in an injectable solution to solubilize the lyophilized decitabine powder just before administration. Delivery of decitabine formulation with cyclodextrins as excipients to a patient may be achieved through all the drug delivery methods, kits, cassettes, and vessels. The great improvement of decitabine solubility and stability in cyclodextrin-containing aqueous solutions would minimize or eliminate a lot of serious inconvenience in both manufacturing and clinical application of current decitabine drug product.

## 3. Comparative Example: Solubility of In Cyclodextrin Solutions

Aqueous solutions of cyclodextrin at pH 6.7-7.2 were prepared by dissolving 4 parts of cyclodextrins (hydroxypropyl $\alpha$-cyclodextrin (HPACD), hydroxypropyl) $\beta$-cycloxdextrin (HPBCD), hydroxypropyl $\gamma$-cyclodextrin (HPGCD), or CAPTISOL) in 6 parts of potasium phosphate buffer (50 mM KH$_2$PO$_4$ pH 7.0), resulting solutions of 40% w/w cyclodoxtrins. Solid 5-azacylidine (Sigma-Aldrich) was added to the aqueous solution of cyclodextrin and mixed at room temperature (20-25°C). **Table 2** list the solubility of 5-azacytidine in each of the cyclodextrin solutions in comparison with that in the potassium phosphate buffer (pH 7.0).

As shown in Table 2, the use of cyclodextrins as excipients in an aqueous solution can increase 5-azacytidine solubility in the solution $\beta$-cyclodextrins such as HPBCD and Captisol are more effective in enhancing 5- azacytidine solubility in aqueous solutions than $\alpha$-, or $\gamma$-cyclodextrins (such as HPBCD and HPGCD). The solubility increase could translate into less volume squired per dosage clinically.

**Table 2**

|  | KPi (pH 7.0) | 4O%HPBCD (w/w,pH7.2) | 40%Captisole (w/w.pH6.7) | 40%HPGCD (w/w,pH7.0) | 40%HPACD (w/w, pH7.0) |
|---|---|---|---|---|---|
| Solubility (mg/mL) | 19 | 26 | 26 | 22 | 20 |

It can be apreciated to one of ordinary skill in the art that many changes and modifications can be made to the instant invention without departing from the spirit or scope of the appended claims and such changes and modifications are contemplated within the scope of the instant invention.

**Claims**

1.  A pharmaceutical composition, comprising:

    decitabine and a cyclodextrin compound solvated in an aqueous solvent comprising at least 60% v/v water.

2.  The pharmaceutical composition of claim 1, wherein decitabine is in the form of pharmaceutically-acceptable salt.

3.  The pharmaceutical composition of claim 2, wherein the pharmaceutically-acceptable salt is selected from the group consisting of hydrochloric, hydrobromic, hydroiodic, nitric, carbonic, sulfuric and phosphoric acid.

4.  The pharmaceutical composition of claim 2, wherein the pharmaceutically-acceptable salt is selected from the group consisting of aliphatic, cycloaliphatic, aromatic, arylaliphatic, heteroclyclic, carboxylic, formic, acetic, propionic, succinic, glycolic, gluconic, maleic, embonic, methansulfonic, ethanesulfonic, 2-hydroxyethanesulfonic, pantothenic, benzenesulfonic, toluenesulfonic, sulfanilic, mesylic, cyclohexylaminosulfonic, stearic, algenic, β-hydroxybutyric, malonic, galactic,galacturonic acid, L-lactic, acetic, (+)-L-tartaric, citric, butyric, hexanoic, L-aspartic, L-glutamic, succinic, EDTA, maleic, methanesulfonic acid, HBr, HF, HI, nitric, nitrous, sulfuric sulfurous, phosphorous, perchloric, chloric, chlorous acid, carboxylic acid, sulfonic acid, ascorbic, carbonic, fumaric acid, ethanesulfonic, 2-hydroxyethanesulfonic, and toluenesulfonic acid.

5.  The pharmaceutical composition of claim 1, wherein the cyclodextrin compound is amorphous or crystalline.

6.  The pharmaceutical composition of claim 1, wherein the cyclodextrin compound is an amorphous α-,β- or γ-cyclodextrin compound,

7.  The pharmaceutical composition of claim 1, wherein the cyclodextrin compound is an alkylated or hydroxyalkyl cyclodextrin compound.

8.  The pharmaceutical composition of claim 1, wherein the cyclodextrin compound is selected from the group consisting of hydroxypropyl, hydroxyethyl, glucosyl, maltosyl and maltotriosyl derivatives of β-cyclodextrin, carboxyamidomethyl-β-cyclodextrin, carboxymethyl-β-cyclodextrin hydroxypropyl-β-cyclodextrin and diethylamino-β-cyclodextrin.

9.  The pharmaceutical composition of claim 1, wherein the cyclodextrin compound is a sulfoalkylether cyclodextrin derivative.

10. The pharmaceutical composition of claim 1, wherein the cyclodextrin compound is selected from the group consisting of mono-,tetra and hepta-substituted β-cyclodextrin sulfobutyl ether sodium salt.

11. The pharmaceutical composition of claim 1, wherein the cyclodextrin compound is β-cyclodextrin sulfobutyl ether 7 sodium salt or CAPTISOL.

12. The pharmaceutical composition of claim 1, wherein the amount of decitabine in the pharmaceutical composition is between 0.1 and 200 mg per ml of solvent.

13. The pharmaceutical composition of claim 1, wherein the of decitabine in the pharmaceutical composition is between 2 and 50 mg per ml of solvent

14. The composition of claim 1, wherein the weight ratio of decitabine to the cyclodextrin compound is in a range between 1:1 and 1:5000.

15. The pharmaceutical composition of claim 1, wherein the weight ratio of decitabine to the cyclodextrin compound is in a range between 1:1 and 1:200.

16. The pharmaceutical composition of claim 1, wherein the weight ratio of decitabine to the cyclodextrin compound is in a range between 1:5 and 1:50.

17. The pharmaceutical composition of claim 1, wherein the aqueous solvent comprise at least 90% water.

18. The pharmaceutical composition of claim 1, wherein the aqueous solvent comprise at least 98% water.

19. The pharmaceutical composition of claim 1, wherein the aqueous solvent further comprises propylene glycol glycerin, polyethylene glycol, or a combination thereof.

20. The pharmaceutical composition of claim 1, further comprising: an acidifying agent added to the composition in a proportion such that the composition has a resulting pH between about 4 and 8.

21. The pharmaceutical composition of claim 20, the acidifying agent is an organic acid.

22. The pharmaceutical composition of claim 21, wherein the organic acid is selected from the group consisting of ascorbic acid, citric acid, tartaric acid, lactic acid, oxalic acid, formic acid, benzene sulphonic acid, benzoic acid, maleic acid, glutamic acid, succinic acid, aspartic acid, diatrizoic acid, and acetic acid.

23. The pharmaceutical composition of claim 20, wherein the agent is an inorganic acid.

24. The pharmaceutical composition of claim 23, wherein the inorganic acid is selected from the group consisting of hydrochloric acid, sulphuric acid phosphoric acid, and nitric acid.

25. The pharmaceutical composition of claim 20, wherein the acidifying agent is ascorbic acid at a concentration of 0.01 -0.2 mg/ml of the solvent

26. The pharmaceutical compositon of claim 1, further comprising an excipient selected to group consisting of sorbitol, lactose, and dextrose.

27. The pharmaceutical composition of claim 1, wherein the composition is at least 80% stable upon storage at 2-8°C for 5, 10, 15, 24, hours, or 2, 4, 7, 14, 21, 28, or more days

28. The pharmaceutical composition of claim 1, wherein the pharmaceutical composition is at least 95% stable upon storage 2-8°C for 5, 10, 15, 24 hours, or 2, 4, 7, 14, 21, 28 or more days.

29. The pharmaceutical composition of claim 1, wherein the composition is at least 80% stable upon storage at 20-25°C for 5, 10, 15, 24 of 2, 5, 14, 21, 28 or more days.

30. The pharmaceutical composition of claim 1, wherein the pharmaceutical composition is at least 95% stable upon storage at 20-25°C for 5, 10, 15, 24 hours, or 2, 5, 7, 14, 21, 28 or more days.

31. A for preparing a pharmaceutical composition to claim 1, said process comprising:

    dissolving decitabine and a cyclodextrin compound in an aqueous solvent that comprises at least 60% v/v water,

32. A process according to claim 31, wherein the aqueous solvent further comprises a buffer salt

33. A process according to claim 32, the buffer salt is potassium phosphate.

34. A process according to claim 31, which further comprises: adding an acidifying agent to the solution containing decitabine and the solvent such that pH of the resulting solution is between pH 4 and pH 8.

35. A process according to claim 31, which further comprises: adding an acidifying agent to the solution containing decitabine and the such that pH of the resulting solution is between pH 5.5 and pH 6.8.

36. A sterilized vessel comprising the pharmaceutical composition of claim 1.

37. The vessel of claim 36, wherein the vessel in a vial, syringe, or ampoule.

38. The vessel of claim 36, wherein the vessel contains between 1 and 50 ml of the pharmaceutical composition.

39. A kit comprising decitabine in a solid form, and an aqueous diluent that comprises a cyclodestrin compound and at least 60% v/v water.

40. The kit of claim 39, wherein decitabine is contained in a first container in the kit and the aqueous diluent is contained in a second container in the kit.

41. A kit comprising:

a first container containing and a cyclodextrin compound; and
a second container an aqueous diluent comprising at least 60% water.

42. kit of claim 41, the aqueous diluent further comprises buffer salt.

43. Use of a pharmaceutical composition according to claim 1 in the preparation of a medicament for treating a disease selected from the group consisting of benign tumours, cancer, hematological disorders, atherosclerosis, insults to body tissue due to surgery, abnormal wound healing, abnormal angiogenesis, that produce fibrosis of tissue, repetitive motion disorders, disorders of tissues that are not highly and proliferative responses associated with organ transplants.

44. Use according to claim 43, the pharmaceutical composition is for administration orally, parenterally, topically, intraperitoneally, intravenously, intraarterially, transdermally, sublingually, intramuscularly, rectally, transbuccally, intranasally, liposomally, via inhalation, vaginally, intraoccularly, via local delivery, subcutaneously, intraadiposally, intraarticularly, or intrathecally.

45. Use to claim 43, the pharmaceutical composition is for administration intravenously, intramuscularly, or subcutaneously.

46. Use according to claim 43, the pharmaceutical composition is further diluted with an aqueous diluent prior to administration to the patient.

47. Use to claim 46, the aqueous diluent is infusion and the diluted pharmaceutical composition is for intravenous infusion,

48. Use according to claim 46, wherein the dilution is performed 10 hr, 2hr, 1 hr, 30 min, 10 min, 5 min or less before to the patient.

49. Use according to claim 43, wherein said medicament is for with a therapeutic agent other than decitabine.

50. Use according to claim 49, the therapeutic agent is selected the group consisting of antimetabolic agents, alkylating agents, retinoid compounds, hormonal agents, plant-derived agents, biologic agents, interleukins, interferons, cytokines, immuno-modulating and monoclonal antibodies.

51. Use according to claim 43, the disease is selected the group or myelodysplastic syndrome, leukaemia, malignant tumours, and sickle-cell anemia.

52. Use according to claim 43, wherein said medicament is prepared by mixing decitabine and a cyclodextrin compound that are in a sold form with an aqueous diluent at least 60% v/v water to form a pharmaceutical formulation.

53. Use according to claim 43, wherein said medicament is prepared by mixing decitabine in a solid form an aqueous diluent comprising a cyclodextrin compound solvated in at least 60% v/v water to form a pharmaceutical formulation.

54. A pharmaceutical composition according to claim 1 for use in treating a disease selected from the group consisting of benign tumours, cancer, hematological disorders, atherosclerosis, insults to body tissue due to surgery, abnormal wound healing, abnormal angiogenesis, diseases that produce fibrosis of tissue, repetitive motion disorders, of tissues are not highly vascularised, and proliferative responses associated with organ transplants.

**Patentansprüche**

1. Pharmazeutische Zusammensetzung, welche umfasst:

Decitabin und eine Cyclodextrinverbindung, solvatisiert in einem wässerigen Lösungsmittel, welches mindestens 60% Vol./Vol. Wasser umfasst.

2. Pharmazeutische Zusammensetzung nach Anspruch 1, worin Decitabin in Form eines pharmazeutisch annehmbaren Salzes ist.

3. Pharmazeutische Zusammensetzung nach Anspruch 2, worin das pharmazeutisch annehmbare Salz ausgewählt ist aus der Gruppe bestehend aus Salz-, Bromwasserstoff-, Iodwasserstoff-, Salpeter-, Kohlen-, Schwefel- und Phosphorsäure.

4. Pharmazeutische Zusammensetzung nach Anspruch 2, worin das pharmazeutisch annehmbare Salz ausgewählt ist aus der Gruppe bestehend aus aliphatischer, cycloaliphatischer, aromatischer, arylaliphatischer, heterocyclischer Säure, Carbon-, Ameisen-, Essig-, Propion-, Bernstein-, Glycol-, Glucon-, Malein-, Embon-, Methansulfon-, Ethansulfon-, 2-Hydroxyethansulfon-, Pantothen-, Benzolsulfon-, Toluolsulfon-, Sulfanil-, Mesyl-, Cyclohexylaminosulfon-, Stearin-, Algen-, β-Hydroxybutter-, Malon-, Galact-, Galacturonsäure, L-Milch-, Essig-, (+)-L-Wein-, Zitronen-, Butter-, Hexan-, L-Asparagin-, L-Glutamin-, Bernstein-, EDTA, Malein-, Methansulfonsäure, HBr, HF, HI, Salpeter-, Salpetrig-, Schwefel-, Schweflig-, Phosphorig-, Perchlor-, Chlor-, Chlorigsäure, Carbonsäure, Sulfonsäure, Ascorbin-, Kohlen-, Fumarsäure, Ethansulfon-, 2-Hydroxyethansulfon- und Toluolsulfonsäure.

5. Pharmazeutische Zusammensetzung nach Anspruch 1, worin die Cyclodextrinverbindung amorph oder kristallin ist.

6. Pharmazeutische Zusammensetzung nach Anspruch 1, worin die Cyclodextrinverbindung eine amorphe α-, β- oder γ-Cyclodextrinverbindung ist.

7. Pharmazeutische Zusammensetzung nach Anspruch 1, worin die Cyclodextrinverbindung eine alkylierte oder Hydroxyalkyl-Cyclodextrinverbindung ist.

8. Pharmazeutische Zusammensetzung nach Anspruch 1, worin die Cyclodextrinverbindung ausgewählt ist aus der Gruppe bestehend aus Hydroxypropyl-, Hydroxyethyl-, Glucosyl-, Maltosyl- und Maltotriosylderivaten von β-Cyclodextrin, Carboxyamidomethyl-β-cyclodextrin, Carboxymethyl-β-cyclodextrin, Hydroxypropyl-β-cyclodextrin und Diethylamino-β-cyclodextrin.

9. Pharmazeutische Zusammensetzung nach Anspruch 1, worin die Cyclodextrinverbindung ein Sulfoalkylethercyclodextrinderivat ist.

10. Pharmazeutische Zusammensetzung nach Anspruch 1, worin die Cyclodextrinverbindung ausgewählt ist aus der Gruppe bestehend aus mono-, tetra- und hepta-substituiertem β-Cyclodextrinsulfobutylether-natriumsalz.

11. Pharmazeutische Zusammensetzung nach Anspruch 1, worin die Cyclodextrinverbindung β-Cyclodextrin-sulfobutylether-7-natriumsalz oder CAPTISOL ist.

12. Pharmazeutische Zusammensetzung nach Anspruch 1, worin die Menge an Decitabin in der pharmazeutischen Zusammensetzung zwischen 0,1 und 200 mg pro ml Lösungsmittel ist.

13. Pharmazeutische Zusammensetzung nach Anspruch 1, worin die Menge an Decitabin in der pharmazeutischen Zusammensetzung zwischen 2 und 50 mg pro ml Lösungsmittel ist.

14. Pharmazeutische Zusammensetzung nach Anspruch 1, worin das Gewichtsverhältnis von Decitabin zur Cyclodextrinverbindung im Bereich zwischen 1:1 und 1:5000 ist.

15. Pharmazeutische Zusammensetzung nach Anspruch 1, worin das Gewichtsverhältnis von Decitabin zur Cyclodextrinverbindung im Bereich zwischen 1:1 und 1:200 ist.

16. Pharmazeutische Zusammensetzung nach Anspruch 1, worin das Gewichtsverhältnis von Decitabin zur Cyclodextrinverbindung im Bereich zwischen 1:5 und 1:50 ist.

17. Pharmazeutische Zusammensetzung nach Anspruch 1, worin das wässerige Lösungsmittel mindestens 90% Wasser umfasst.

18. Pharmazeutische Zusammensetzung nach Anspruch 1, worin das wässerige Lösungsmittel mindestens 98% Wasser umfasst.

19. Pharmazeutische Zusammensetzung nach Anspruch 1, worin das wässerige Lösungsmittel ferner Propylenglycol, Glycerin, Polyethylenglycol oder eine Kombination davon umfasst.

20. Pharmazeutische Zusammensetzung nach Anspruch 1, welche ferner ein Säuerungsmittel umfasst, zugegeben zur Zusammensetzung in einem solchen Anteil, dass die Zusammensetzung einen sich ergebenden pH zwischen etwa 4 und 8 hat.

21. Pharmazeutische Zusammensetzung nach Anspruch 20, worin das Säuerungsmittel eine organische Säure ist.

22. Pharmazeutische Zusammensetzung nach Anspruch 21, worin die organische Säure ausgewählt ist aus der Gruppe bestehend aus Ascorbinsäure, Zitronensäure, Weinsäure, Milchsäure, Oxalsäure, Ameisensäure, Benzolsulfonsäure, Benzoesäure, Maleinsäure, Glutaminsäure, Bernsteinsäure, Asparaginsäure, Amidotrizoesäure und Essigsäure.

23. Pharmazeutische Zusammensetzung nach Anspruch 20, worin das Säuerungsmittel eine anorganische Säure ist.

24. Pharmazeutische Zusammensetzung nach Anspruch 23, worin die anorganische Säure ausgewählt ist aus der Gruppe bestehend aus Salzsäure, Schwefelsäure, Phosphorsäure und Salpetersäure.

25. Pharmazeutische Zusammensetzung nach Anspruch 20, worin das Säuerungsmittel Ascorbinsäure bei einer Konzentration von 0,01-0,2 mg/ml des Lösungsmittels ist.

26. Pharmazeutische Zusammensetzung nach Anspruch 1, welche ferner einen Arzneimittelträger umfasst, ausgewählt aus der Gruppe bestehend aus Mannitol, Sorbitol, Lactose und Dextrose.

27. Pharmazeutische Zusammensetzung nach Anspruch 1, wobei die pharmazeutische Zusammensetzung bei Lagerung bei 2-8°C für 5, 10, 15, 24 Stunden oder 2, 4, 7, 14, 21, 28 oder mehr Tage mindestens 80% stabil ist.

28. Pharmazeutische Zusammensetzung nach Anspruch 1, wobei die pharmazeutische Zusammensetzung bei Lagerung bei 2-8°C für 5, 10, 15, 24 Stunden oder 2, 4, 7, 14, 21, 28 oder mehr Tage mindestens 95% stabil ist.

29. Pharmazeutische Zusammensetzung nach Anspruch 1, wobei die pharmazeutische Zusammensetzung bei Lagerung bei 20-25°C für 5, 10, 15, 24 Stunden oder 2, 5, 7, 14, 21, 28 oder mehr Tage mindestens 80% stabil ist.

30. Pharmazeutische Zusammensetzung nach Anspruch 1, wobei die pharmazeutische Zusammensetzung bei Lagerung bei 20-25°C für 5, 10, 15, 24 Stunden oder 2, 5, 7, 14, 21, 28 oder mehr Tage mindestens 95% stabil ist.

31. Verfahren zum Herstellen einer pharmazeutischen Zusammensetzung gemäß Anspruch 1, wobei besagtes Verfahren umfasst: Lösen von Decitabin und einer Cyclodextrinverbindung in einem wässerigen Lösungsmittel, das mindestens 60% Vol./Vol. Wasser umfasst.

32. Verfahren gemäß Anspruch 31, wobei das wässerige Lösungsmittel ferner ein Puffersalz umfasst.

33. Verfahren gemäß Anspruch 32, wobei das Puffersalz Kaliumphosphat ist.

34. Verfahren gemäß Anspruch 31, welches ferner umfasst: Zugeben eines Säuerungsmittels zur Lösung, welche Decitabin und das Lösungsmittel enthält, so dass der pH der sich ergebenden Lösung zwischen pH 4 und pH 8 ist.

35. Verfahren gemäß Anspruch 31, welches ferner umfasst: Zugeben eines Säuerungsmittels zur Lösung, welche Decitabin und das Lösungsmittel enthält, so dass der pH der sich ergebenden Lösung zwischen pH 5,5 und pH 6,8 ist.

36. Sterilisiertes Gefäß, welches die pharmazeutische Zusammensetzung nach Anspruch 1 umfasst.

37. Gefäß nach Anspruch 36, wobei das Gefäß eine Phiole, Spritze oder Ampulle ist.

38. Gefäß nach Anspruch 36, wobei das Gefäß zwischen 1 und 50 ml der pharmazeutischen Zusammensetzung umfasst.

39. Kit, welches Decitabin in einer festen Form und ein wässeriges Verdünnungsmittel umfasst, das eine Cyclodextrinverbindung und mindestens 60% Vol./Vol. Wasser umfasst.

40. Kit nach Anspruch 39, worin Decitabin in einem ersten Behälter im Kit enthalten ist und das wässerige Verdünnungsmittel in einem zweiten Behälter im Kit enthalten ist.

41. Kit, welches umfasst:

   einen ersten Behälter, welcher Decitabin und eine Cyclodextrinverbindung enthält; und
   einen zweiten Behälter, welcher ein wässeriges Verdünnungsmittel enthält, welches mindestens 60% Wasser umfasst.

42. Kit nach Anspruch 41, worin das wässerige Verdünnungsmittel ferner Puffersalz umfasst.

43. Verwendung einer pharmazeutischen Zusammensetzung gemäß Anspruch 1 in der Herstellung eines Medikaments zum Behandeln einer Krankheit, ausgewählt aus der Gruppe bestehend aus gutartigen Tumoren, Krebs, hämatologischen Störungen, Atherosklerose, Insults des Körpergewebes wegen Chirurgie, abnormer Wundheilung, abnormer Angiogenese, Krankheiten, die Fibrose von Gewebe erzeugen, repetitiven Bewegungsstörungen, Störungen von Geweben, die nicht hochgradig vaskularisiert sind, und proliferativen Antworten in Verbindung mit Organtransplantationen.

44. Verwendung gemäß Anspruch 43, wobei die pharmazeutische Zusammensetzung für Verabreichung oral, parenteral, topisch, intraperitoneal, intravenös, intraarteriell, transdermal, sublingual, intramuskulär, rektal, transbukkal, intranasal, liposomal, über Inhalation, vaginal, intraokular, über lokale Abgabe, subkutan, intraadiposal, intraartikulär oder intrathekal ist.

45. Verwendung gemäß Anspruch 43, wobei die pharmazeutische Zusammensetzung für Verabreichung intravenös, intramuskulär oder subkutan ist.

46. Verwendung gemäß Anspruch 43, wobei die pharmazeutische Zusammensetzung vor Verabreichung an den Patienten mit einem wässerigen Verdünnungsmittel weiter verdünnt wird.

47. Verwendung gemäß Anspruch 46, wobei das wässerige Verdünnungsmittel Infusionsflüssigkeit ist und die verdünnte pharmazeutische Zusammensetzung für intravenöse Infusion ist.

48. Verwendung gemäß Anspruch 46, wobei die Verdünnung 10 Std., 2 Std., 1 Std., 30 Min., 10 Min., 5 Min. oder weniger vor Verabreichung an den Patienten durchgeführt wird.

49. Verwendung gemäß Anspruch 43, wobei besagtes Medikament zur Verabreichung mit einem anderen therapeutischen Agens als Decitabin ist.

50. Verwendung gemäß Anspruch 49, wobei das therapeutische Agens ausgewählt ist aus der Gruppe bestehend aus antimetabolischen Agenzien, Alkylierungsmitteln, Retinoidverbindungen, hormonalen Mitteln, von Pflanzen abgeleiteten Agenzien, biologischen Agenzien, Interleukinen, Interferonen, Zytokinen, immunmodulierenden Agenzien und monoklonalen Antikörpern.

51. Verwendung gemäß Anspruch 43, wobei die Krankheit ausgewählt ist aus der Gruppe bestehend aus myelodysplastischem Syndrom, Leukämie, malignen Tumoren und Sichelzellenanämie.

52. Verwendung gemäß Anspruch 43, wobei besagtes Medikament hergestellt wird durch Mischen von Decitabin und einer Cyclodextrinverbindung, die in fester Form sind, mit einem wässerigen Verdünnungsmittel, welches mindestens 60% Vol./Vol. Wasser umfasst, um eine pharmazeutische Formulierung zu bilden.

53. Verwendung gemäß Anspruch 43, wobei besagtes Medikament hergestellt wird durch Mischen von Decitabin in einer festen Form mit einem wässerigen Verdünnungsmittel, welches eine Cyclodextrinverbindung umfasst, solvatisiert in mindestens 60% Vol./Vol. Wasser, um eine pharmazeutische Formulierung zu bilden.

**54.** Pharmazeutische Zusammensetzung gemäß Anspruch 1 zur Verwendung beim Behandeln einer Krankheit, ausgewählt aus der Gruppe bestehend aus gutartigen Tumoren, Krebs, hämatologischen Störungen, Atherosklerose, Insults des Körpergewebes wegen Chirurgie, abnormer Wundheilung, abnormer Angiogenese, Krankheiten, die Fibrose von Gewebe erzeugen, repetitiven Bewegungsstörungen, Störungen von Geweben, die nicht hochgradig vaskularisiert sind, und proliferativen Antworten in Verbindung mit Organtransplantationen.

**Revendications**

**1.** Composition pharmaceutique, 1 comprenant :

de la décitabine et un composé de cyclodextrine solvaté dans un solvant aqueux comprenant au moins 60 % d'eau volume/volume.

**2.** Composition pharmaceutique selon la revendication 1, dans laquelle la décitabine est sous la forme d'un sel acceptable sur le plan pharmaceutique.

**3.** Composition pharmaceutique selon la revendication 2, dans laquelle le sel acceptable sur le plan pharmaceutique est choisi dans le groupe constitué de l'acide chlorhydrique, bromhydrique, iodhydrique, nitrique, carbonique, sulfurique et phosphorique.

**4.** Composition pharmaceutique selon la revendication 2, dans laquelle le sel acceptable sur le plan pharmaceutique est choisi dans le groupe constitué d'un acide aliphatique, cycloaliphatique, aromatique, arylaliphatique, hétérocyclique, carboxylique, formique, acétique, propionique, succinique, glycolique, gluconique, maléique, embonique, méthanesulfonique, éthanesulfonique, 2-hydroxyéthanesulfonique, pantothénique, benzènesulfonique, toluènesulfonique, sulfanilique, mésylique, cyclohexylaminosulfonique, stéarique, algénique, β-hydroxybutyrique, malonique, galactique, galacturonique, L-lactique, acétique, (+)-L-tartrique, citrique, butyrique, hexanoïque, L-aspartique, L-glutamique, succinique, EDTA, maléique, méthanesulfonique, HBr, HF, HI, l'acide nitrique, nitreux, sulfurique, sulfureux, phosphoreux, perchlorique, chlorique, chloreux, un acide carboxylique, l'acide sulfonique, ascorbique, carbonique, fumarique, éthanesulfonique, 2-hydroxyéthanesulfonique, et toluènesulfonique.

**5.** Composition pharmaceutique selon la revendication 1, dans laquelle le composé de cyclodextrine est amorphe ou cristallin.

**6.** Composition pharmaceutique selon la revendication 1, dans laquelle le composé de cyclodextrine est un composé d'α-, β- ou γ-cyclodextrine amorphe.

**7.** Composition pharmaceutique selon la revendication 1, dans laquelle le composé de cyclodextrine est un composé de cyclodextrine alkylée ou hydroxyalkylée.

**8.** Composition pharmaceutique selon la revendication 1, dans laquelle le composé de cyclodextrine est choisi dans le groupe constitué de dérivés d'hydroxypropyle, hydroxyéthyle, glucosyle, maltosyle et maltotriosyle de β-cyclodextrine, carboxyamidométhyl-β-cyclodextrine, carboxyméthyl-β-cyclodextrine, hydroxypropyle-β-cyclodextrine et diéthylamino-β-cyclodextrine.

**9.** Composition pharmaceutique selon la revendication 1, dans laquelle le composé de cyclodextrine est un dérivé de sulfoalkyléther cyclodextrine.

**10.** Composition pharmaceutique selon la revendication 1, dans laquelle le composé de cyclodextrine est choisi dans le groupe constitué de sel de sodium d'éther sulfobutylique de β-cyclodextrine mono-, tétra- et hepta-substituée.

**11.** Composition pharmaceutique selon la revendication 1, dans laquelle le composé de cyclodextrine est le sel de sodium d'éther sulfobutylique 7 de β-cyclodextrine ou CAPTISOL.

**12.** Composition pharmaceutique selon la revendication 1, dans laquelle la quantité de décitabine dans la composition pharmaceutique est comprise entre 0,1 et 200 mg par mL de solvant.

**13.** Composition pharmaceutique selon la revendication 1, dans laquelle la quantité de décitabine dans la composition pharmaceutique est comprise entre 2 et 50 mg par mL de solvant.

14. Composition pharmaceutique selon la revendication 1, dans laquelle le rapport pondéral de décitabine sur le composé de cyclodextrine est dans un intervalle compris entre 1:1 et 1:5000.

15. Composition pharmaceutique selon la revendication 1, dans laquelle le rapport pondéral de décitabine sur le composé de cyclodextrine est dans un intervalle compris entre 1:1 et 1:200.

16. Composition pharmaceutique selon la revendication 1, dans laquelle le rapport pondéral de décitabine sur le composé de cyclodextrine est dans un intervalle compris entre 1:5 et 1:50.

17. Composition pharmaceutique selon la revendication 1, dans laquelle le solvant aqueux comprend au moins 90 % d'eau.

18. Composition pharmaceutique selon la revendication 1, dans laquelle le solvant aqueux comprend au moins 98 % d'eau.

19. Composition pharmaceutique selon la revendication 1, dans laquelle le solvant aqueux comprend en outre du propylène glycol, de la glycérine, du polyéthylène glycol, ou une de leur combinaison.

20. Composition pharmaceutique selon la revendication 1, comprenant en outre : un agent acidifiant ajouté à la composition en une proportion telle que la composition a un pH résultant compris entre environ 4 et 8.

21. Composition pharmaceutique selon la revendication 20, dans laquelle l'agent acidifiant est un acide organique.

22. Composition pharmaceutique selon la revendication 21, dans laquelle l'acide organique est choisi dans le groupe constitué de l'acide ascorbique, l'acide citrique, l'acide diacétyltartrique, l'acide lactique, l'acide oxalique, l'acide formique, l'acide benzène-sulfonique, l'acide benzoïque, l'acide maléique, l'acide glutamique, l'acide succinique, l'acide aspartique, l'acide diatrizoïque et l'acide acétique.

23. Composition pharmaceutique selon la revendication 20, dans laquelle l'agent acidifiant est un acide inorganique.

24. Composition pharmaceutique selon la revendication 23, dans laquelle l'acide inorganique est choisi dans le groupe constitué de l'acide chlorhydrique, l'acide sulfurique, l'acide phosphorique et l'acide nitrique.

25. Composition pharmaceutique selon la revendication 20, dans laquelle l'agent acidifiant est l'acide ascorbique à une concentration de 0,01 à 0,2 mg/mL du solvant.

26. Composition pharmaceutique selon la revendication 1, comprenant en outre un excipient choisi dans le groupe constitué du mannitol, sorbitol, lactose et dextrose.

27. Composition pharmaceutique selon la revendication 1, où la composition pharmaceutique est stable à au moins 80 % lors du stockage de 2 à 8 °C pendant 5, 10, 15, 24 heures ou 2, 4, 7, 14, 21, 28 jours ou plus

28. Composition pharmaceutique selon la revendication 1, où la composition pharmaceutique est stable à au moins 95 % lors du stockage de 2 à 8 °C pendant 5, 10, 15, 24 heures ou 2, 4, 7, 14, 21, 28 jours ou plus

29. Composition pharmaceutique selon la revendication 1, où la composition pharmaceutique est stable à au moins 80 % lors du stockage de 20 à 25 °C pendant 5, 10, 15, 24 heures ou 2, 5, 7, 14, 21, 28 jours ou plus

30. Composition pharmaceutique selon la revendication 1, où la composition pharmaceutique est stable à au moins 95 % lors du stockage de 20 à 25 °C pendant 5, 10, 15, 24 heures ou 2, 5, 7, 14, 21, 28 jours ou plus

31. Procédé de préparation d'une composition pharmaceutique selon la revendication 1, ledit procédé comprenant :

   la dissolution de décitabine et d'un composé de cyclodextrine dans un solvant aqueux qui comprend au moins 60 % d'eau volume/volume.

**32.** Procédé selon la revendication 31, dans lequel le solvant aqueux comprend en outre un sel tampon.

**33.** Procédé selon la revendication 32, dans lequel le sel tampon est du phosphate de potassium.

**34.** Procédé selon la revendication 31, qui comprend en outre : l'ajout d'un agent acidifiant à la solution contenant la décitabine et le solvant de telle sorte que le pH de la solution résultante est compris entre pH 4 et pH 8.

**35.** Procédé selon la revendication 31, qui comprend en outre : l'ajout d'un agent acidifiant à la solution contenant la décitabine et le solvant de telle sorte que le pH de la solution résultante est compris entre pH 5,5 et pH 6,8.

**36.** Récipient stérilisé comprenant la composition pharmaceutique selon la revendication 1.

**37.** Récipient selon la revendication 36, où le récipient est un flacon, une seringue ou une ampoule.

**38.** Récipient selon la revendication 36, où le récipient contient entre 1 et 50 mL de la composition pharmaceutique.

**39.** Trousse comprenant : de la décitabine sous une forme solide, et un diluant aqueux qui comprend un composé de cyclodextrine et au moins 60 % d'eau volume/volume.

**40.** Trousse selon la revendication 39, dans laquelle la décitabine est contenue dans un premier récipient dans la trousse, et le diluant aqueux est contenu dans un deuxième récipient dans la trousse.

**41.** Trousse comprenant :

un premier récipient contenant de la décitabine et un composé de cyclodextrine ; et
un deuxième récipient contenant un diluant aqueux comprenant au moins 60 % d'eau.

**42.** Trousse selon la revendication 41, dans laquelle le diluant aqueux comprend en outre un sel tampon.

**43.** Utilisation d'une composition pharmaceutique selon la revendication 1, pour le traitement d'une maladie choisie dans le groupe constitué de tumeurs bénignes, cancer, troubles hématologiques, athérosclérose, agressions à un tissu du corps du fait d'une intervention chirurgicale, cicatrisation anormale, angiogenèse anormale, maladies qui produisent une fibrose de tissu, troubles répétitifs du mouvement, troubles de tissus qui ne sont pas fortement vascularisés, et réponses prolifératives associées à des greffes d'organe.

**44.** Utilisation selon la revendication 43, dans laquelle la composition pharmaceutique est destinée à une administration par voie orale, par voie parentérale, localement, par voie intrapéritonéale, par voie intraveineuse, par voie intra-artérielle, par voie transcutanée, par voie sublinguale, par voie intramusculaire, par voie rectale, par voie buccale, par voie intranasale, par voie liposomique, par inhalation, par voie vaginale, par voie intra-oculaire, par le biais d'une libération locale, par voie sous-cutanée, par voie intra-adipeuse, par voie intra-articulaire, ou par voie intrathécale.

**45.** Utilisation selon la revendication 43, dans laquelle la composition pharmaceutique est destinée à une administration par voie intraveineuse, par voie intramusculaire ou par voie sous-cutanée.

**46.** Utilisation selon la revendication 43, dans laquelle la composition pharmaceutique est en outre diluée avec un diluant aqueux et administrée au patient.

**47.** Utilisation selon la revendication 46, dans laquelle le diluant aqueux est un fluide de perfusion et la composition pharmaceutique diluée est destinée à une perfusion intraveineuse.

**48.** Utilisation selon la revendication 46, dans laquelle la dilution est effectuée 10 h, 2 h, 1 h, 30 min, 10 min, 5 min ou moins avant administration au patient.

**49.** Utilisation selon la revendication 43, dans laquelle ledit médicament est destiné à une administration avec un agent thérapeutique autre que la décitabine.

**50.** Utilisation selon la revendication 54, dans laquelle l'agent thérapeutique est choisi dans le groupe constitué d'agents antimétaboliques, agents d'alkylation, composés de rétinoïde, agents hormonaux, agents végétaux, agents biologiques, interleukines, interférons, cytokines, agents d'immunomodulation, et anticorps monoclonaux.

51. Utilisation selon la revendication 43, dans laquelle la maladie est choisie dans le groupe constitué de syndrome myélodysplasique, leucémie, tumeurs malignes, et anémie drépanocytaire.

52. Utilisation selon la revendication 43, dans laquelle ledit médicament est préparé en mélangeant la décitabine et un composé de cyclodextrine qui sont sous une forme solide avec un diluant aqueux comprenant au moins 60 % d'eau volume/volume de façon à former une formulation pharmaceutique ;

53. Utilisation selon la revendication 43, dans laquelle ledit médicament est préparé en mélangeant la décitabine sous une forme solide avec un diluant aqueux comprenant un composé de cyclodextrine solvaté dans au moins 60 % d'eau volume/volume de façon à former une formulation pharmaceutique ;

54. Composition pharmaceutique selon la revendication 1, destinée à être utilisée pour le traitement d'une maladie choisie dans le groupe constitué de tumeurs bénignes, cancer, troubles hématologiques, athérosclérose, agressions à un tissu du corps du fait d'une intervention chirurgicale, cicatrisation anormale, angiogenèse anormale, maladies qui produisent une fibrose de tissu, troubles répétitifs du mouvement, troubles de tissus qui ne sont pas fortement vascularisés, et réponses prolifératives associées à des greffes d'organe.

**FIGURE 1**

EP 1 819 227 B1

FIGURE 2

EP 1 819 227 B1

FIGURE 3

**FIGURE 4**

29

FIGURE 5

EP 1 819 227 B1

y = 977.84x + 22.509
R² = 0.9939

1/delta A

1/[HPBCD] (mM⁻¹)

**FIGURE 6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4727064 A, Pitha

**Non-patent literature cited in the description**

- **Pompon et al.** *J. Chromat,* 1987, vol. 388, 113-122
- **Kissinger ; Stenm.** *J. Chromat.,* 1986, vol. 353, 309-318
- **Groeningen CJ et al.** *Cancer Res.,* 1986, vol. 46, 4831-4836
- **Chabot GG ; Momparler RL.** Proceedings of the Workshop on 5-aza-2'-deoxycytidine. PCH Publication, 1990, 105-115
- **Momparler et al.** *MOL. PHARMACOL.,* 1985, vol. 30, 287-299
- **Bouchard ; Mompaler.** *Mol. Pharmacol.,* 1983, vol. 24, 109-114
- **Juttermann et al.** *Proc. Natl. Acad. Sci. USA,* 1994, vol. 91, 11797-11801
- **Santini et al.** *Ann. Int. Med.,* 2001, vol. 134, 573-588